(19) Europäisches Patentamt
European Patent Office
Office européen des brevets

(11) EP 1 560 837 B1

# (12) FASCICULE DE BREVET EUROPEEN

(45) Date de publication et mention de la délivrance du brevet:
**05.08.2009 Bulletin 2009/32**

(21) Numéro de dépôt: **03782533.8**

(22) Date de dépôt: **07.11.2003**

(51) Int Cl.:
***C07H 15/18*** *(2006.01)* ***A61K 31/7016*** *(2006.01)*
***A61P 25/00*** *(2006.01)* ***C07C 323/47*** *(2006.01)*

(86) Numéro de dépôt international:
**PCT/FR2003/003335**

(87) Numéro de publication internationale:
**WO 2004/043982 (27.05.2004 Gazette 2004/22)**

## (54) NOUVEAUX DERIVES AMPHIPHILES DE L ALPHA-C-PHENYL-N-TERT-BUTYL NITRONE

AMPHIPHILEN VON ALFA-C-PHENYL-N-TERT-BUTYL NITRONEN

NOVEL AMPIPHILIC DERIVATIVES OF ALPHA-C-PHENYL N-TERT-BUTYL NITRONE

(84) Etats contractants désignés:
**AT BE BG CH CY CZ DE DK EE ES FI FR GB GR HU IE IT LI LU MC NL PT RO SE SI SK TR**

(30) Priorité: **08.11.2002 FR 0214078**

(43) Date de publication de la demande:
**10.08.2005 Bulletin 2005/32**

(73) Titulaires:
- **TS Pharma**
  **13510 Eguilles (FR)**
- **Universite D'Avignon et Des Pays Du Vaucluse**
  **84029 Avignon Cedex 1 (FR)**

(72) Inventeurs:
- **DURAND, Grégory**
  **F-30401 Villeneuve Lez Avignon Cedex (FR)**
- **POLIDORI, Ange**
  **F-84000 Avignon (FR)**
- **PUCCI, Bernard**
  **F-13940 Molleges (FR)**

(74) Mandataire: **Corizzi, Valérie et al**
**Cabinet ORES,**
**36, rue de Saint Pétersbourg**
**75008 Paris (FR)**

(56) Documents cités:
**WO-A-91/05552** **US-A- 5 455 272**

- **O. OUARI ET AL.: "Synthesis of a glycolipidic amphiphilic nitrone as a new spin trap" J. ORG. CHEM., vol. 64, 1999, pages 3554-3556, XP002250820**
- **FLOYD R A ET AL: "PROTECTION AGAINST OXIDATIVE DAMAGE TO CNS BY ALPHA-PHENYL-TERT-BUTYL NITRONE AND OTHER SPIN-TRAPPING AGENTS: A NOVEL SERIES OF NONLIPID FREE RADICAL SCAVENGERS" EMERGING STRATEGIES IN NEUROPROTECTION, XX, XX, 1992, pages 252-272, XP000992315**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

L'invention a pour objet de nouveaux composés, dérivés de l'α-C-phényl-*N*-*tert*-butyl nitrone, un procédé pour leur préparation et leur utilisation pour la préparation de médicaments destinés à prévenir ou traiter les maladies liées au stress oxydatif.

Les pathologies liées au stress oxydatif et à la formation des espèces radicalaires oxygénées ont été répertoriées par Croos C.E., Arch. Intern. Med. (1987) 107, 526-545 et par Anderson K.M., Ells G., Bonomi P., Harris J.E., Medical Hypotheses, (1999) 52, 53-57.

Elles sont nombreuses : plus de 70 pathologies de ce type sont citées dans cette liste qui inclut notamment les maladies immunitaires et inflammatoires, le syndrome d'ischémie-reperfusion, l'athérosclérose, les maladies d'Alzheimer et de Parkinson, les lésions dues aux radiations UV et ionisantes, certaines formes de carcinogenèse chimique ainsi que le vieillissement cellulaire.

Les espèces radicalaires oxygénées et nitrogénées (ROS et RNS) sont produites naturellement dans l'organisme et leur régulation est assurée par certaines enzymes spécialisées comme la Super Oxyde Dismutase soluble (SODs). Le piégeage de ces espèces radicalaires extrêmement réactives est vital car elles occasionnent des dégâts irréversibles dans la cellule. Si la production normale de ces espèces radicalaires est régulée facilement par la cellule, une surproduction de radicaux libres liée à un stress oxydatif externe (choc inflammatoire, syndrome d'ischémie-reperfusion, ...) ou une déficience génétique (anomalie mitochondriale notamment) entraîne une dégradation cellulaire rapide. La prise en charge de cet important flux de radicaux par l'organisme humain ou animal devient dès lors impossible.

Il existe plusieurs mécanismes de défense contre le stress oxydatif de la cellule susceptibles de s'exercer à différents niveaux de la cascade oxydative. Celle-ci est initiée généralement par la surproduction de radicaux superoxyde liée à une réduction partielle de l'oxygène moléculaire dans la mitochondrie (syndrome typique de l'ischémie-reperfusion). Ce radical superoxyde peut se dismuter en eau oxygénée. Ces deux espèces, par l'intermédiaire de la réaction de Fenton, en présence de fer ferreux, peuvent donner des radicaux hydroxyle qui ont la particularité de réagir très rapidement et de manière non spécifique avec n'importe lequel des composants de la cellule comme les lipides, l'ADN ou les protéines, causant des dommages irréversibles parmi ceux-ci, comme cela a été décrit par Stadtman H.R., Berlett B.S. J. Biol. Chem. (1991) 266, 17201-17211 ; Floyd R.A. Carcinogenesis (1990) 11, 1447-1450 ; Gille J.J., Van Berkel C.G., Joenge H. Carcinogenesis (1994) 15, 2695-2699 ; Halliwell B. Mutat. Res. (1999) 443, 37-52.

Ces espèces radicalaires en activant certains gènes suicides (gènes Bel ou p53) par l'intermédiaire du facteur NF-kB sont également à l'origine du phénomène d'apoptose cellulaire qui a été décrit par Siebenlist U., Franzoso G., Brown K. Annu. Rev. Cell. Biol. (1994) 10, 405-455.

La SOD soluble est chargée de convertir le radical superoxyde en eau oxygénée, celle-ci étant ensuite prise en charge par des catalases des peroxydases glutathion dépendantes.

Il existe d'autres niveaux de protection cellulaires contre les oxydants notamment au niveau de la membrane, qui permettent de limiter l'oxydation des phospholipides membranaires insaturés. L'α-tocophérol et le β-carotène sont les principaux exemples d'antioxydants lipidiques.

La stratégie la plus prometteuse, dans la recherche d'une thérapie destinée à prévenir ou traiter les maladies liées au stress oxydatif, consiste à intervenir le plus en amont possible de cette cascade oxydative, afin de prévenir très tôt les dommages liés à la très forte réactivité des espèces radicalaires.

Pour cela on a cherché à piéger ces radicaux libres hautement réactifs par l'intermédiaire de molécules dites « spin-trap » ou « piège à spin », parmi lesquelles les nitrones apparaissent comme les plus efficaces.

L'effet thérapeutique des nitrones dans la réduction et la prévention des dommages causés par les radicaux libres dans les systèmes biologiques a été mis en évidence en 1990 par Oliver C., Starke-Read P., Stadman E., Liu G., Carney J., Floyd R. Proc. Natl. Acad. Sci. USA (1990) 87, 5144-5147.

Ces auteurs ont pu démontrer une diminution des dommages causés par une ischémie cérébrale après injection de l'α-C-phényl-*N*-*tert*-butyl nitrone (PBN) chez les gerbilles. Les ischémies cérébrales sont accompagnées d'une forte augmentation de la production de radicaux libres qui ont été piégés par la PBN pour former des adduits de spin bien plus stables donc moins réactifs et toxiques. La PBN est le spin trap qui a fait l'objet du plus grand nombre d'études biologiques.

On peut par exemple se reporter à Hensley K., Carney J.M., Stewart C.A., Tabatabaie T., Pye Q.N., Floyd R.A. Int. Rev. Neurobiol. (1997) 40, 229-317.

Elle possède une grande spécificité d'action cérébrale probablement en raison de son hydrophobie importante qui lui permet de franchir la barrière hématoméningée, comme cela a été montré par Cheng H.Y., Liu T., Feuerstein G., Barone F.C. Free Radic. Biol. Med (1993) 14, 243-250.

Parmi les nitrones, les plus connus et les plus efficaces sont les α-C-phényl-*N*-*tert*-butylnitrone (PBN), les 5,5-diméthylpyrrolidine-*N*-oxide (DMPO) et des molécules découvertes plus récemment: la *N*-benzylidène-1-diéthoxyphosphoryl-1-méthyléthylamine *N*-oxyde (PBNP) et la 5-diéthyl-phosphono 5-méthyl pyrroline-*N*-oxyde (DEPMPO).

On peut également citer un dérivé disulfonate de la PBN, le NXY-059 (disodium 4-[(tert-butylimino)-méthylbenzene-1,3-disulfonate *N*-oxyde) possédant une activité neuroprotectrice supérieure à la PBN et qui est en cours d'étude pharmacologique et de développement clinique :

Kuroda S., Tsuchidate R., Smith M.L., Maples K.R., Siesjo B.K. J. Cereb. Blood Flow Metab. (1999) 19, 778-787 ; Lees K.R., Sharma A.K., Barer D., Ford G.A., Kostulas V., Cheng Y.F., Odergren T. Stroke (2001) 32, 675-680.

Toutefois, aucune des molécules citées ci-dessus ne possède une efficacité *in vivo* ou *ex vivo* satisfaisante à faible dose, même si leur concentration cytotoxique est très élevée : Almli L.M., Hamrick S.E.G., Koshy A.A., Täuber M.G., Ferriero D.M. Dev. Brain Res. (2001) 132, 121-129 ; Nakao N., Grasbon-Frodl E.M., Widner H., Brundin P. Neuroscience (1996) 73, 185-200. Ce manque d'efficacité est probablement lié à une mauvaise biodisponibilité de la drogue et à un problème de pénétration cellulaire.

Il subsiste donc le besoin d'une molécule de type spin trap, ou piège à spin, capable de piéger des radicaux libres, et qui soit également susceptible d'être acheminée par l'organisme humain ou animal jusqu'à sa cible au niveau intracellulaire.

En particulier, une molécule capable de traverser la membrane cellulaire et, challenge encore plus important et difficile, la membrane mitochondriale afin d'entrer dans le compartiment où est produit le radical superoxyde.

Dans ce but, Ouari O., Polidori A., Pucci B., Tordo P., Chalier F. J. Org. Chem. (1999) 64, 3554-3556 et Geromel V., Kadhom N., Cebalos-Picot I., Ouari O., Polidori A., Munnich A., Rötig A., Rustin P. Hum. Mol. Genet. (2001) 10, 1221-1228, ont proposé un dérivé amphiphile perfluorocarboné de la PBN : la TA1PBN.

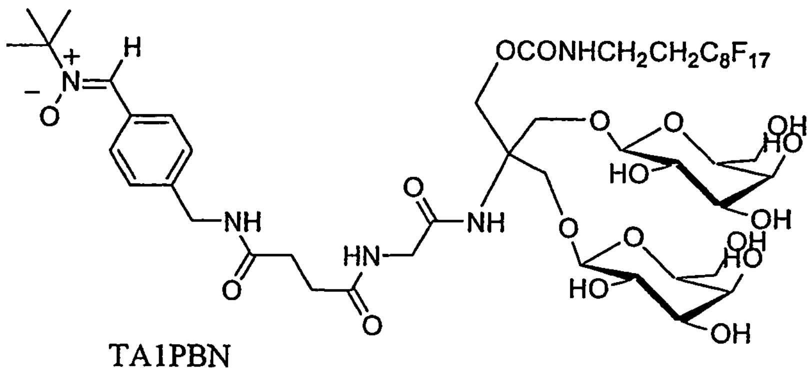

TA1PBN

Cette molécule a été testée sur des lignées cellulaires de fibroblastes souffrant d'un sévère déficit d'activité du complexe V de la chaîne respiratoire (ATPase) et elle a donné des résultats encourageants.

Cependant, la synthèse de la TA1PBN présente des difficultés qui rendent sa production à l'échelle industrielle difficilement envisageable.

Aussi, la Demanderesse s'est fixée pour objectif la conception et la fabrication de nouvelles molécules, ayant une activité de spin trap ou piège à spin, présentant une biodisponibilité accrue par rapport aux molécules de l'art antérieur et dont la préparation soit simple et permette d'envisager une production à l'échelle industrielle.

L'invention a pour objet de nouvelles molécules, caractérisées en ce qu'elles répondent à la formule (I) ci-dessous :

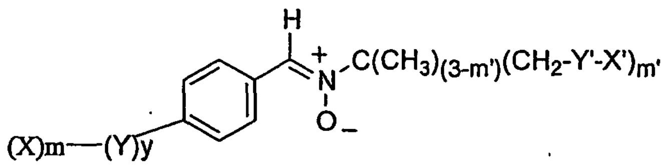

(I)

dans laquelle :

X représente un groupement hydrophile choisi parmi un mono ou un polysaccharide, ainsi que les dérivés aminés de mono et de polysaccharides, une chaîne polyoxyde d'éthylène, une chaîne peptidique, un groupement polaire

ionique choisi parmi un ammonium quaternaire, un oxyde d'amine, un groupement carnitine ;
m représente un entier égal à 1, 2 ou 3 ;
Y représente un bras espaceur destiné à relier le noyau aromatique et les substituants hydrophiles X ;
Y est choisi parmi les fonctions ester, amide, urée, uréthane, éther, thio-éther, amine, les chaînes hydrocarbonées en $C_1$-$C_6$, éventuellement interrompues par une ou plusieurs fonctions ester, amide, urée, uréthane et par un ou plusieurs ponts éther, amine ou thio-éther ;
y représente un entier égal à 0 ou à 1 ;
$Y^1$ représente un groupement choisi Parmi une fonction ester

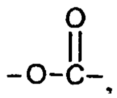

une fonction amide

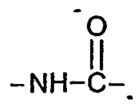

une fonction urée

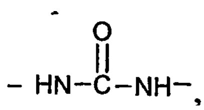

une fonction uréthane

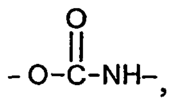

un pont éther -O-, un pont thio-éther -S- ;
m' est un entier choisi parmi 1 et 2 ;
X' représente une chaîne alkyle en $C_4$-$C_{14}$ éventuellement substituée par un ou plusieurs atomes de fluor.

Parmi les mono-saccharides utilisables dans la présente invention, on peut citer : le glucose, le lactose, le fructose, le mannose, le galactose, le ribose, le maltose. Parmi les dérivés aminés de sucres, on peut citer notamment la glucosamine. Parmi les polysaccharides utilisables dans la présente invention, on peut citer les chaînes constituées de plusieurs unités monosaccharide comme par exemple : le saccharose et le lactobionamide.

Lorsque la partie hydrophile X de la molécule de formule (I) est une chaîne polyoxyde d'éthylène, celle-ci comprend avantageusement de 30 à 100 unités oxyde d'éthylène, de préférence de 50 à 60 unités.

De préférence la chaîne peptidique est constituée d'acides aminés naturels comme l'alanine, l'arginine, l'asparagine, l'acide aspartique, la cystéine, la glutamine, l'acide glutamique, la glycine, l'histidine, l'isoleucine, la leucine, la lysine, la méthionine, la phénylalanine, la proline, la sérine, la thréonine, le tryptophane, la tyrosine, la valine.

Des groupements hydrophiles ioniques ou non ioniques utilisables dans la présente invention sont illustrés dans le schéma 1 ci-dessous.

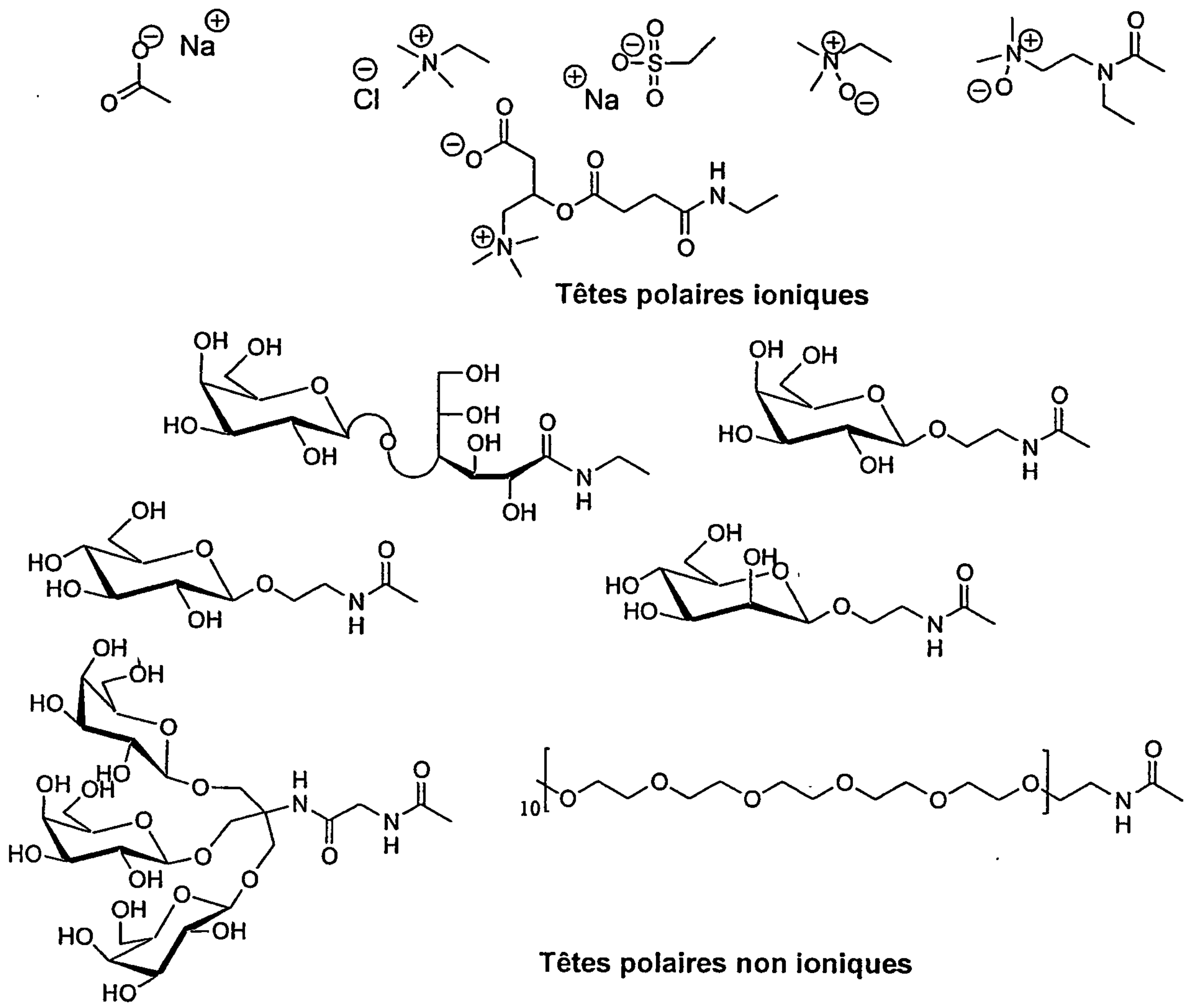

Têtes polaires ioniques

Têtes polaires non ioniques

Schéma 1 : structure générale des têtes polaires

En fonction de la mono ou de la plurifonctionnalité du bras espaceur Y, celui-ci est substitué une ou deux fois par le groupement X.

Le groupement X' peut par exemple être choisi parmi les radicaux suivants :

- radicaux hydrocarbonés : le n-butyle, le ter-butyle, l'isobutyle, le n-pentyle, l'isopentyle, le n-hexyle, le n-heptyle, le n-octyle, le n-nonyle, le n-décyle, le n-undécyle, le n-dodécyle, le n-tridécyle, le n-tétradécyle...
- radicaux hydrocarbonés fluorés : on peut citer ceux répondant à la formule $-(CH_2)_t(CF_2)_rF$, dans laquelle r et t représentent deux entiers avec : $14 \geq r+t \geq 4$, tels que par exemple :
- $(CF_2)_4F$ ; $-(CF_2)_5F$ ; $-(CF_2)_6F$; $-(CF_2)_7F$; $-(CF_2)_8F$ ; $-(CF_2)_9F$; - $(CF_2)_{10}F$ ; $-(CF_2)_{11}F$ ; $-(CF_2)_{12}F$ ; $-(CF_2)_{13}F$; $-(CF_2)_{14}F$ ; $-CH_2-(CF_2)_3F$ ; $-CH_2-(CF_2)_4F$ ; - $CH_2-(CF_2)_5F$; $-CH_2-(CF_2)_6F$; $-CH_2-(CF_2)_7F$; $-CH_2-(CF_2)_8F$; $-CH_2-(CF_2)_9F$ ; $-CH_2-(CF_2)_{10}F$;- $CH_2-(CF_2)_{11}F$; $-CH_2-(CF_2)_{12}F$; $-CH_2-(CF_2)_{13}F$; $-(CH_2)_2-(CF_2)_2F$; $-(CH_2)_2-(CF_2)_3F$; $-(CH_2)_2-(CF_2)_4F$ ; $-(CH_2)_2-(CF_2)_5F$ ; $-(CH_2)_2-(CF_2)_6F$ ; $-(CH_2)_2-(CF_2)_7F$ ; $-(CH_2)_2-(CF_2)_8F$; $-(CH_2)_2-(CF_2)_9F$; $-(CH_2)_2-(CF_2)_{10}F$; $-(CH_2)_2-(CF_2)_{11}F$; $-(CH_2)_2-(CF_2)_{12}F$; - $(CH_2)_3-(CF_2)_1F$ ;............... $-(CH_2)_{13}-(CF_2)F$.

De façon préférentielle, l'une ou moins des conditions ci-dessous est satisfaite :

X représente un groupement lactobionamide, carnitine ou une chaîne polyoxyéthylène ;
m représente 1 ;
m' représente 1 ou 2 ;
X' est choisi parmi octyl, décyl, dodécyl, $CF_3(CF_2)_rCH_2CH_2$-

8≥r≥6

Les composés de l'invention présentent l'avantage, par rapport aux composés de l'art antérieur, d'être dotés d'une biodisponibilité améliorée. Cette biodisponibilité améliorée est au moins en partie attribuable au caractère amphiphile des molécules de l'invention.

L'invention a également pour objet un procédé de préparation des composés répondant à la formule (I), ce procédé étant caractérisé en ce que l'on fait réagir un aldéhyde répondant à la formule (II) avec une hydroxylamine répondant à la formule (III) suivant le schéma 2 ci-dessous :

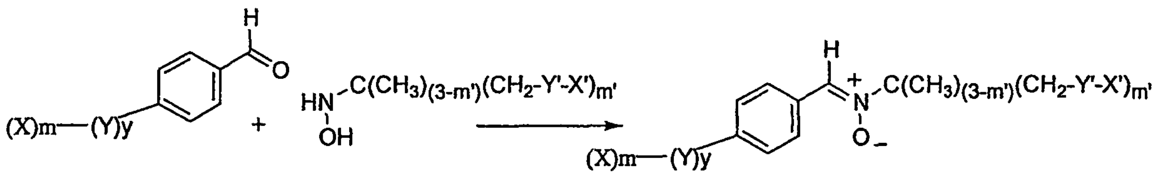

(II) (III) (I)

Schéma 2

dans lequel X, y, Y, m, X', m' et Y' ont la même définition que ci-dessus.

Les composés de formule (III) sont préparés suivant un procédé décrit dans le schéma 3 ci-dessous :

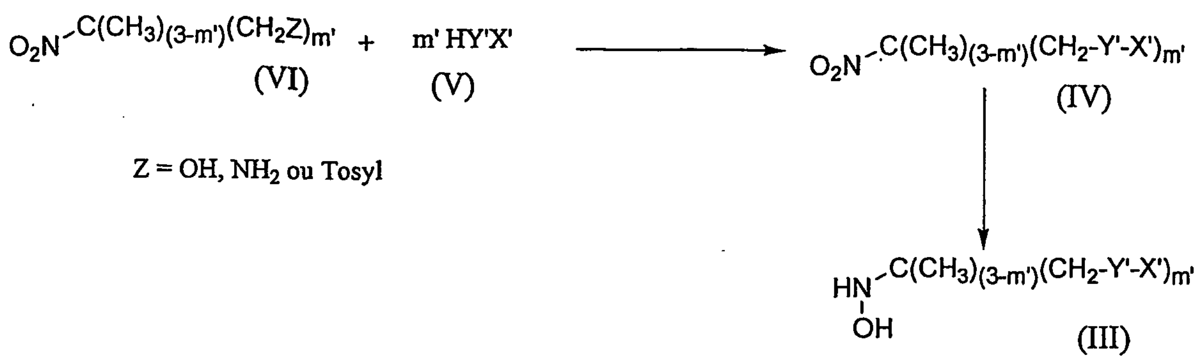


(VI) (V) (IV) (III)

Schéma 3

En fonction de la nature du groupement lipophile, le schéma 3 est mis en oeuvre dans des conditions qui seront exposées ci-dessous.

**a- Partie hydrophobe monocaténaire hydro ou perfluorocarbonée (Figure 1)** :

La figure 1 illustre la préparation des composés de formule (III) avec :

m'=1;
X' = $(CH_2)_2$-R avec R = $C_6F_{13}$, $C_8F_{17}$, $CH_3(CH_2)_n$
4<n<14 ;

Y' = 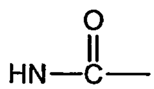

(composé 5),

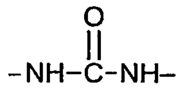

(composé 6),

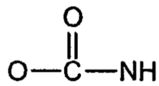

(composé 2),

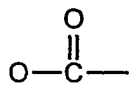

(composé 1), -S-(composé 7) ;

La partie hydrophobe monocaténaire est synthétisée à partir du 2-méthyl 2-nitro propanol. La fonction alcool de ce synthon permet de greffer les chaînes hydro et perfluorocarbonées directement par l'intermédiaire de liaisons ester par réaction de l'alcool et de l'acide en présence d'un agent de couplage, la dicyclohexylcarbodiimide et de dimethyl aminopyridine **(1).**

L'alcool peut également réagir avec un isocyanate d'alkyle, pour donner des liaisons de type uréthane **(2).**

La fonction alcool peut être convertie en amine par tosylation suivie d'une substitution par l'azoture de sodium. Par réaction de Staudinger, l'azoture d'alkyle est transformé en amine en présence de triphényl phosphine et de soude.

Cette amine peut réagir avec un acide gras pour donner une liaison de type amide **(5)** ou avec un isocyanate d'alkyle pour former une urée **(6).**

Enfin, le tosylate peut être substitué en milieu basique par un thiol pour former une liaison thioéther **(7).**

La fonction nitro des différents synthons hydrophobes **(1-7)** est ensuite réduite en hydroxylamine par l'intermédiaire de 4 équivalents du réactif de Kagan (SmI2) dans un mélange THF/McOH ou dans l'acide acétique.

Cette réaction a été décrite par Girard P., Namy J.L., Kagan H.B. J. Am. Chem. Soc. (1980) 102, 2693-2698 et Namy J.L., Girard P, Kagan H.B. Nouv. J. Chem. (1977) 1, 5.

La réaction très rapide (3min.) est réalisée avec un rendement variant entre 50 et 100% selon la nature du nitroalkyle à réduire.

**b- Partie hydrophobe bicaténaire hydro ou perfluorocarbonée (Figure 2) :**

La figure 2 illustre la préparation des composés de formule (III) avec :

m'=2;
X' = $(CH_2)_2$-R avec R = $C_6F_{13}$, $C_8F_{17}$, $CH_3(CH_2)_n$
4<n<14;

Y' = 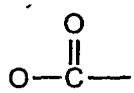

(composé **8**),

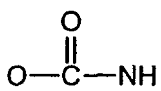

(composé **9**), -S- (composé **12**),

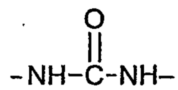

(composé **14**),

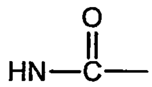

(composé **13**).

Les parties hydrophobes bicaténaires sont synthétisées à partir du 2-nitro-2-méthyl-1,3-propanediol. Les chaînes grasses sont fixées sur les fonctions alcool par des liaisons uréthane **(9)** ou ester **(8).** Les fonctions alcool sont transformées en tosylate par réaction sur le chlorure de tosyle. Le ditosylate peut être substitué par un alkyl mercaptan pour donner un thioéther **(12).** Ce ditosylate peut être transformé en diamine par substitution du tosylate par l'azoture de sodium et réaction avec la triphényl phosphine et hydrolyse basique. Cette diamine peut réagir avec un isocyanate pour donner une liaison urée **(14)** ou un acide pour former une liaison amide **(13)**.

La fonction nitro des synthons biantennés est ensuite réduite par le réactif de Kagan avec un rendement pouvant varier de 60 à 80% en fonction de la molécule concernée.

**c- Partie hydrophile non ionique (Figure 3) :**

La figure 3 illustre la préparation des composés de formule (II) dans laquelle :

X représente un groupement polaire non ionique ;
Y représente NH-$CH_2$- (composé **20**),

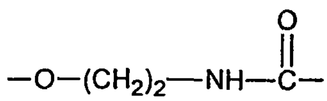

(composés **21, 22, 23**),

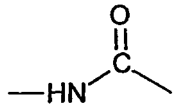

(composé **24)**,

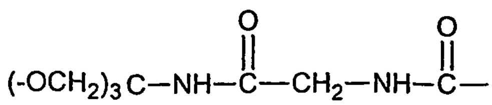

(composé **25**) ;
y=1 ;
m = 1 (composés **20** à **24)** ;
m = 3 (composé **25)**

Les têtes hydrophiles non ioniques sont constituées de sucres (lactobionamide, galactose, glucose, mannose...), de polyol glycosylés ou non (tels que le Tris par exemple...) ou de polyéthylène glycol. Les dérivés de la lactobionamide **20** sont synthétisés à partir du 4-cyano benzaldéhyde et de la lactobionolactone. Après protection de la

fonction aldéhyde par acétalisation (**15)** puis réduction du groupement nitro, l'aminé obtenue est condensée sur la lactobionolactone. L'acétylation des fonctions alcool et la déprotection de la fonction aldéhyde par l'acétaldéhyde en excès en milieu acide donne le synthon polaire **20.**

Les autres têtes polaires sont synthétisées à partir du 4-carboxybenzaldéhyde. Les dérivés glucosylés **(21),** mannosylés **(22),** galactosylés **(23),** sont obtenus par condensation du Boc aminoéthanol sur les acétobromoglycosides correspondants **(17, 18, 19)** dans les conditions de la réaction d'Helferich. Après déprotection de la fonction amine et condensation sur la fonction acide, en présence d'un agent de couplage peptidique on obtient les 3 têtes polaires glycosylées **21-23.**

Le dérivé pegylé **24** est issu de la condensation d'un polyéthylène glycol fonctionnalisé par une amine sur la fonction acide du 4-carboxybenzaldéhyde protégé par un acétal. La déprotection de l'acétal permet l'obtention de ce dérivé. Enfin, il est possible d'obtenir un dérivé trigalactosylé **25** par condensation d'une amine déjà décrite dans la littérature par Polidori A., Pucci B., Zarif L., Lacombe J-M., Riess J-G., Pavia A.A., Chem. Phys. Lipids (1995) 77, 225-251, sur la fonction acide du 4-carboxybenzaldéhyde.

**d- Partie hydrophile ionique (Figure 4) :**

La figure 4 illustre la préparation des composés de formule (II) dans laquelle :

X représente un groupement polaire ionique ;
Y représente $-CH_2-$ (composés **26, 27**),

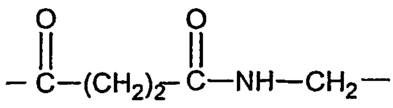

(composé **28**),

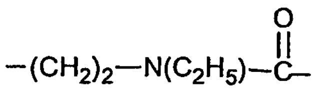

(composé **29)** ;
y=1;
m = 1.

Les têtes polaires ioniques sont constituées de groupements ammonium quaternaire, oxyde d'amine ou carnitine. Le groupement ammonium est synthétisé à partir du nitrile après réduction par $AlLiH_4$ et protection préalable de la fonction aldéhyde par l'éthylène glycol. L'amine obtenue est perméthylée par l'iodure de méthyle en présence de tributylamine dans le DMF, selon la méthode décrite par Sommer H.Z., Lipp H.I, Jackson L.L. J. Org. Chem. (1971) 36, 824-828.

Le produit cristallisé est hydrolysé dans l'acide acétique aqueux de manière à récupérer le dérivé **26.**

L'oxyde d'amine **27** est obtenu à partir de la même amine après formation de l'aminé tertiaire en présence de 2 équivalents d'iodure de méthyle. L'azote est oxydée par de l'eau oxygénée, 10 volumes dans le méthanol. Après déprotection de l'acétal on obtient le composé **27.**

L'oxyde d'aminé **29** est synthétisé par la méthode de McQuade et al. A partir du 4-carboxybenzaldéhyde protégé sur sa fonction aldéhyde par un groupement acétal et de la N-éthyl N', N'-diméthyl éthylène diamine. Cette méthode a été décrite par McQuade D.T., Quinn M.A., YU S.M., Polans A.S., Krebs M.P., Gellman S.H. Angew. Chem. Int. Ed. (2000) 39, 758-761.

Le couplage est réalisé en présence d'un agent de couplage peptidique, la DCC. Après oxydation de la fonction amine par l'eau oxygénée, 10 volumes et déprotection de l'acétal on récupère le composé **29.**

Enfin, le dérivé **28** de la carnitine est obtenu par condensation de l'aminé **15** sur l'anhydride succinique puis couplage de la fonction acide sur la fonction alcool de la carnitine dans le DMF en présence de DCC. Après déprotection de la fonction cétal, on obtient le produit **28.**

**e- Obtention des nitrones amphiphiles mono (Figure 5) et bicaténaires (Figure 6) :**

Les différentes nitrones amphiphiles sont obtenues par couplage de la fonction aldéhyde des différents synthons polaires sur le groupement hydroxylamine des parties hydrophobes. En fonction de la nature plus ou moins polaire des têtes hydrophiles ioniques (très polaires), notées I, ou non ioniques glycosylées (apolaires car acétylées), notées NI, on utilisera un solvant polaire protique (éthanol) ou aprotique (THF). La réaction est cependant plus rapide dans les solvants polaires protiques (2 jours au lieu de 10 dans le THF).

Le rectangle portant l'indication CH représente, dans ces figures, la chaîne hydrocarbonée éventuellement fluorée X'.

Le THF est utilisé avec des têtes polaires glycosylées car c'est un solvant qui ne provoque pas de réaction de desacétylation des fonctions alcool. Toutes les nitrones amphiphiles glycosylées ont été purifiées par CLHP en phase inverse (colonne C18/éluant méthanol-eau). Les composés ioniques ont été isolés par cristallisation. Les nitrones amphiphiles pegylées sont purifiées par chromatographie d'exclusion (sephadex LH20).

L'invention a en outre pour objet l'utilisation des composés répondant à la formule (I) telle que définie ci-dessus comme agent anti-radicaux libres.

En effet, il a été démontré que les composés selon la présente invention étaient dotés d'une capacité à piéger les radicaux libres équivalente à celle des composés de l'art antérieur.

Cette propriété permet d'envisager l'utilisation des molécules de l'invention dans des domaines variées :

- dans le domaine thérapeutique, les produits de l'invention peuvent être employés pour la prévention et/ou le traitement des pathologies liées au stress oxydatif et à la formation des espèces radicalaires oxygénées.

L'invention a par conséquent pour objet les compositions pharmaceutiques comprenant un composé selon l'invention dans un support pharmaceutiquement acceptable. Elle a pour objet l'utilisation d'un composé selon l'invention pour la préparation d'un médicament destiné à prévenir et/ou traiter les effets des radicaux libres.

Elle a également pour objet l'utilisation d'un composé de l'invention pour la préparation d'une composition pharmaceutique destinée à prévenir et/ou traiter les pathologies liées au stress oxydatif et à la formation des espèces radicalaires oxygénées, notamment les maladies immunitaires et inflammatoires, le syndrome d'ischémie-reperfusion, l'athérosclérose, la maladie d'Alzeimer, la maladie de Parkinson, la maladie de Huntington, les lésions dues aux radiations UV et ionisantes, les cancers, le vieillissement cellulaire.

Les produits de l'invention peuvent être administrés par toute voie connue de l'homme du métier, notamment par injection intraveineuse ou intramusculaire, par administration orale ou cutanée. Ils peuvent être utilisés seuls ou en association avec d'autres actifs. Leur dosage et la quantité administrée quotidiennement sont adaptés en fonction de l'activité mesurée pour la molécule concernée et en fonction du poids du patient.

- dans le domaine cosmétique, les composés de l'invention peuvent être utilisés pour prévenir et/ou traiter les effets du vieillissement ainsi que les effets des radiations solaires.

L'invention a donc également pour objet une composition cosmétique comprenant un composé de l'invention dans un support cosmétiquement acceptable.

Ladite composition peut être destinée à une application sur la peau ou sur les phanères (ongles, cheveux).

Elle peut se présenter sous forme d'une solution aqueuse ou huileuse, d'une émulsion eau-dans-l'huile ou huile-dans-l'eau, d'une émulsion triple, d'un onguent.

Les composés de l'invention peuvent être introduits dans toute composition cosmétique pour laquelle une activité antiradicalaire est recherchée : une crème de soin cutanée, un produit de protection solaire, un démaquillant, un masque pour la peau ou les cheveux, un shampoing, un produit de maquillage tel qu'un rouge à lèvres, un fard, un fond de teint, un vernis à ongles, etc...

- dans le domaine de la synthèse organique, les composés de l'invention peuvent être utilisés comme capteurs de radicaux libres dans des réactions radicalaires.

Du fait de leur solubilité dans des milieux variés, les composés de l'invention sont faciles à mettre en oeuvre et peuvent être employés dans des conditions très diverses.

## PARTIE EXPERIMENTALE

### I- Evaluation biologique:

Le composé **$A_1$** a été utilisé pour mettre en oeuvre des expériences de piégeage de radicaux libres. Plusieurs composés selon l'invention ont été testés *in vitro* pour leur capacité biologique antioxydante et antiradicalaire.

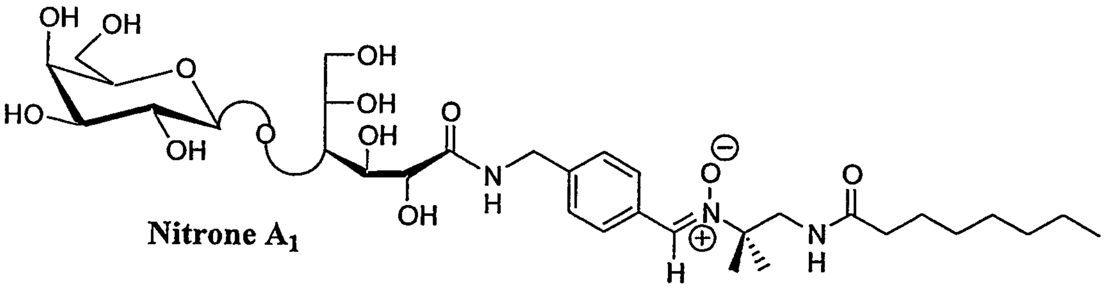

Nitrone $A_1$

### 1- Mesure de la capacité de piégeage des espèces radicalaires

Les expériences de piégeage de radicaux libres centrés sur le carbone (radicaux $CH_3$ et $CO_2$ et sur l'oxygène (radical OH) réalisés sur le composé **$A_1$** ont démontré que la fonctionnalisation de la PBN n'affectait pas la capacité de ces molécules à piéger les espèces radicalaires. Dans le cas des radicaux libres centrés sur le carbone des signaux RPE caractéristiques de ces espèces radicalaires ont pu être observés comme cela est illustré par la figure 7.

En revanche, lors de la génération de radicaux hydroxylés dans le système, des signaux RPE caractéristiques du piégeage de radicaux centrés sur le carbone sont détectés. Cela est dû au piégeage par la nitrone de radicaux carbonés produits sur les têtes polaires par réaction des radicaux OH avec les hydrogènes des sucres.

### 2- Mesure *in vitro* de la capacité biologique antioxydante et antiracalaire

#### a- Evaluation de la capacité antiapoptotique sur neurones corticaux de rats par dosage de l'activité enzymatique de la caspase III.

Ces tests préliminaires ont été réalisés sur un dérivé nitrone amphiphile glycosylé hydrocarboné : la nitrone $A_2$. Son activité anti apoptotique a été comparée à deux nitrones commerciales, la PBN et la DMPO.

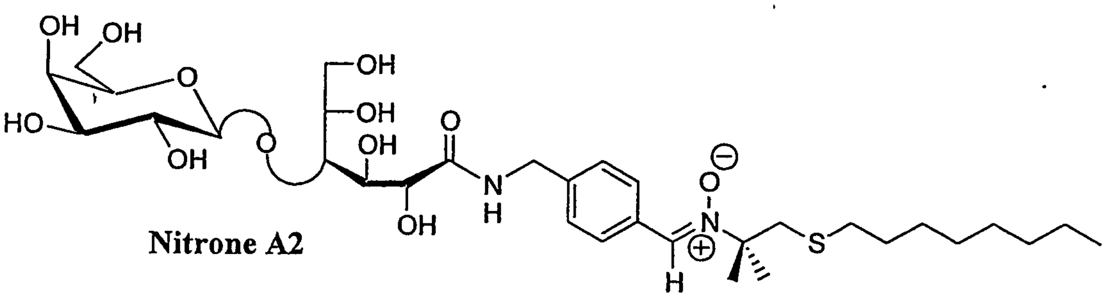

Nitrone A2

Les cellules neuronales de rat ont été intoxiquées pendant 20 min par de l'eau oxygénée à 100 μM au 8ème jour de culture. Cette addition d'eau oxygénée crée un phénomène d'apoptose comme cela a été décrit par Whittemore E.R., Loo D.T., Cotman C.W. Neuroreport (1994) 5, 1485-1488 (vérifié par un contrôle positif d'apoptose par addition de staurosporine) évalué par dosage colorimétrique à 405 nm d'une enzyme spécifique de ce métabolisme, la caspase III par rapport à un témoin d'intoxication maximale (précedemment décrit par Nicholson D.W., Ali A., Thombury N.A., Vaillancourt J.P., Ding C.H., Gallant M., Griffm P.R., Labelle M., Lazebnik Y.A., Munday N.A., Raju S.M., Smulson M.E., Yannin T., Yu V.I., Miller D.K. Nature (1995) 376, 37-43.)

Les différentes molécules à tester ont été incubées pendant 20 heures à des concentrations variables non toxiques (10, 100, 200 μM) avant intoxication par l'eau oxygénée. Après rinçage et séchage à l'étuve les cellules sont

lysées avant dosage colorimétrique. La nitrone amphiphile **$A_2$** possède une cytotoxicité significative à partir de 400 μM.

Les résultats obtenus (illustrés par la figure 8) montrent clairement une très nette diminution de l'activité caspase III après intoxication à l'eau oxygénée 100 μM en présence de la nitrone amphiphile **$A_2$.** Cette activité se révèle beaucoup plus faible que l'activité normale de la caspase III sur des cellules neuronales non intoxiquées. Les résultats indiquent clairement un niveau de protection supérieur à celui mesuré pour les nitrones commerciales PBN et DMPO.

**b- Evaluation de l'efficacité neuroprotectrice sur cocultures nerf-muscle**

L'évaluation protectrice de ces nitrones amphiphiles a été mesurée sur des cocultures nerfs-muscles après intoxication 30 min à l'eau oxygénée.

Les cellules musculaires humaines issues de prélèvements de muscles striés sains sont isolées par migration de cellules satellites dans un milieu de culture approprié. Ces cellules vont fusionner en myofibres non contractiles dans le milieu de culture. Des explants de moelle épinière d'embryon de rat sont déposés sur les cellules musculaires.

Après trois semaines, toutes les fibres musculaires au voisinage des explants se contractent et possèdent des jonctions neuromusculaires matures. Après maturation, ces cultures sont sélectionnées et filmées avec une caméra vidéo couplée à un microscope. Les molécules de type **A ($A_1$, $A_2$, $A_3$, $A_4$)** et **B ($B_1$)** sont incubées pendant 20h00 aux concentrations de 100 et 200 μM. Les cellules sont ensuite intoxiquées pendant 30 min avec $H_2O_2$ 800 mM puis rincées. 24h00 et 48h00 après avoir généré ce stress oxydatif, les cellules sont observées et filmées.

Après 20h00 d'incubation il s'avère que le composé **$B_1$** ionique de type carboxylate est cytotoxique et provoque une dégradation rapide des cellules musculaires. Les autres composés ne sont pas toxiques mais provoquent un arrêt ou un ralentissement des contractions musculaires quelque soit la contraction utilisée (100 et 200 μM). En revanche, on observe une récupération totale ou partielle des contractions 48h00 après intoxication par l'eau oxygénée pour les composés perfluorés **$A_3$** et **$A_4$** à la concentration de 100 μM et hydrocarbonés **$A_1$** et **$A_2$** à 100 et 200 μM (tableau 1). Les autres composés protègent les cellules de la dégradation mais ne permettent pas la récupération des contractions.

La quantification de l'état d'apoptose a ensuite été réalisée après lyse des cellules et centrifugation en dosant la quantité d'ADN fragmentée par un kit « cell death detection ELISA » dans les surnageants. Après révélation enzymatique, les densités optiques sont mesurées à 405 nm par un lecteur de plaque (figure 9).

Les résultats indiquent clairement que toutes les molécules testées, à l'exclusion du dérivé ionique **B1,** protègent les cellules de l'apoptose induite par l'addition d'eaux oxygénée. A une concentration de 200 mM, le dérivé carboxylate **B1** présente un signal d'apoptose très faible artefactuel qui est du avant la lyse cellulaire à une libération des fragments d'ADN dans le milieu de culture après la mort des cellules en culture traitées par cette nitrone.

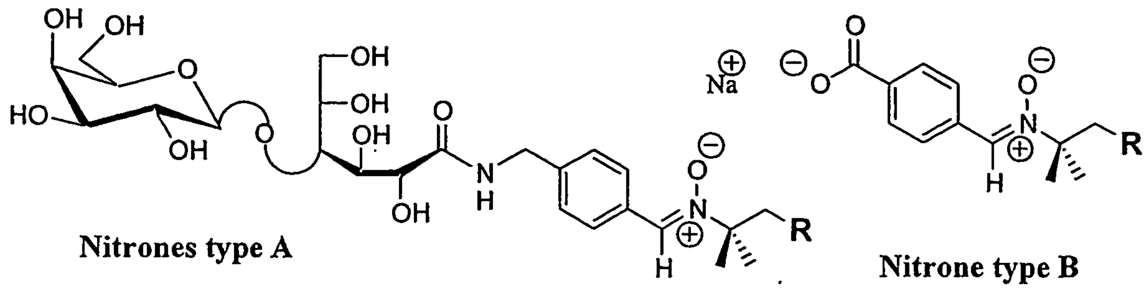

**Nitrones type A**

**Nitrone type B**

**Tableau 1 :** Mesure de l'activité contractile de cultures cellulaires nerf-muscle 48h00 après intoxication par $H_2O_2$ 800 μM

| Nitrones | R | [C] μM | Activité contractile des fibres musculaires(nombres de puits) | | | | % d'inactivité contractile |
|---|---|---|---|---|---|---|---|
| | | | 0 | + | ++ | +++ | |
| **$A_1$** | $C_7H_{15}CONH$ | 100 | | | 2 | | 0 |
| | | 200 | | 3 | | | 0 |
| **$A_2$** | $C_8H_{17}S$ | 100 | 3 | | | | 100 |
| | | 200 | 2 | | | | 100 |

(suite)

| Nitrones | R | [C] $\mu M$ | Activité contractile des fibres musculaires(nombres de puits) | | | | % d'inactivité contractile |
|---|---|---|---|---|---|---|---|
| | | | 0 | + | ++ | +++ | |
| **$A_3$** | $C_6F_{13}CH_2CH_2S$ | 100 | | | | 3 | 0 |
| | | 200 | 1 | 1 | | 1 | 33.3 |
| **$A_4$** | $C_6F_{13}CH_2CH_2CONH$ | 100 | 1 | 2 | | | 33.3 |
| | | 200 | 3 | | | | 100 |
| **$B_1$** | $C_8H_{17}S$ | 100 | mc[1] | | | | 100 |
| | | 200 | mc | | | | 100 |
| PBN | | 200 | mc | | | | 100 |
| DMPO | | 200 | mc | | | | 100 |
| Témoin | | | | | 1 | 1 | 0 |
| Témoin $H_2O_2$ | | | | 3 | 1 | | 75 |
| [1] mort cellulaire | | | | | | | |

**Tableau 2** : Critère d'observation de l'activité contractile des fibres musculaires

| | |
|---|---|
| 0 | Pas de fibres musculaires ayant une activité contractile dans le puits de culture |
| 0/+ | Une fibre musculaire ayant une activité contractile faible et irrégulière |
| + | Une fibre musculaire ayant une activité contractile régulière |
| ++ | 2 à 4 fibres musculaires ayant une activité contractile |
| +++ | Plus de 4 fibres musculaires ayant une activité contractile |

**c. Evaluation de l'activité antioxydante sur des lignées cellulaires de fibroblastes souffrant d'un sévère déficit du complexe V de la chaîne respiratoire: détermination de la viabilité cellulaire par le test MTT**

Les tests sont réalisés sur des lignées cellulaires de fibroblastes caractérisées par une mutation du gène NARP codant pour une protéine (sous-unité 6) du complexe V de la chaîne mitochondriale. Ces cellules sont caractérisées par une surproduction anormale de l'enzyme superoxyde dismutase suggérant que ce déficit génétique provoque un accroissement de la production de radical superoxyde. Cette surproduction de radical superoxyde provoque un processus d'apoptose accéléré des cellules (Geromel V., Kadhom N., Cebalos-Picot I., Ouari O., Polidori A., Munnich A., Rötig A., Rustin P. Hum. Mol. Genet. (2001) 10, 1221-1228).

Des cultures de fibroblastes ont été préparées à partir d'une biopsie de peau de deux individus (contrôle) et d'un patient porteur de la mutation NARP. Les cellules ont été cultivées dans un milieu RPMi 1640 (commercialisé par Life technologies SARL, Cergy Pontoise, France) additionné de glutamax (446 mg/l), 10% sérum foetal de veau non dialysé, 100 $\mu$g/ml streptomycine, 100 IU/ml pénicilline, 200 $\mu$M uridine, 2.5 mM pyruvate de sodium. Pour les tests de cytotoxicité les cellules ont été ensemencées à une densité de 3000 cellules par puits dans des microboites de Petri à 37°C sous 5% de $CO_2$. Pour initier le stress oxydatif, la cellule est soumise après 24 heures à une hypoglycémie par remplacement du glucose par du galactose à 10 mM (milieu sélectif noté ms dans les figures 10a à 10f). Après 24 heures les cellules ont été exposées pendant 48 heures et 72 heures à des concentrations croissantes des différents composés à tester en milieu sélectif destiné aux cellules respiratoires (milieu RPMi 1640 sans glucose). Aux fins de comparaison, toutes les études ont été faites sur des cellules recueillies après un même doublement de population..

L'activité antioxydante des nitrones amphiphiles a été évaluée en mesurant leur capacité à protéger les cellules contre l'apoptose avec le test MTT.

Le test MTT est une méthode colorimétrique qui permet de déterminer le nombre de cellules viables en essais de prolifération et de cytotoxicité. Les puits ont été mis à incuber avec 20 $\mu$l d'une solution de MTT (5 mg/ml dans du PBS) pendant 1 heure à 37°C. Puis, 200 $\mu$l d'isopropanol ont été ajoutés pour extraire le MTT formazan et l'absorbance de chaque puit a été mesuré à 540 nm à l'aide d'un appareil de lecture automatique.

Les résultats obtenus avec les essais colorimétriques MTT sont illustrés par les figures 10a à 10f. Dans ces figures le composé $A_5$ est un nitrone de type A dans lequel $R=OCONH(CH_2)_5CH_3$ et le composé H répond à la formule ci-dessous :

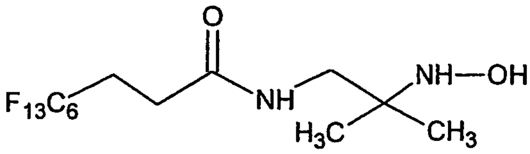

Composé H

Ce test démontre la capacité de protection de la TA1PBN dès la concentration de 50 μM à protéger les cellules NARP de la mort cellulaire par apoptose. Ces résultats confirment donc bien les analyses précédemment effectuées sur la TA1PBN (Geromel V., Kadhom N., Cebalos-Picot I., Ouari O., Polidori A., Munnich A., Rötig A., Rustin P. Hum. Mol. Genet. (2001) 10, 1221-1228). Le composé $\mathbf{B_1}$ perfluorocarboné semble également être efficace à partir d'une concentration de 100 μM. Il est à noter que le composé perfluorocarboné $\mathbf{A_4}$ et les composés hydrocarbonés $\mathbf{A_1}$, $\mathbf{A_2}$ et $\mathbf{A_5}$ ne sont pas efficaces sur ce modèle cellulaire. On peut imputer ce défaut d'efficacité à un manque d'hydrophobie des chaînes grasses hydrocarbonées. Le composé perfluoré $\mathbf{A_4}$ possède une longueur de chaîne inférieure à celle du composé $\mathbf{A_3}$. Il est à noter enfin que la TA1PBN présente une chaîne perfluorée en $C_8F_{17}$ plus longue que celle du composé $\mathbf{A_3}$ (chaîne en $C_6F_{13}$). Ceci peut expliquer la différence d'efficacité dans le traitement des cellules NARP par ces deux nitrones amphiphiles. Des tests sont en cours sur des dérivés analogues à $\mathbf{A_3}$ possédant une chaîne fluorée en $C_8F_{17}$. En conclusion il semble que le degré d'hydrophobie de ces nitrones semble jouer un rôle crucial sur leur activité biologique. Elle doit probablement être conditionnée par la capacité de transfert de la nitrone à travers la membrane cytoplasmique et peut-être dans la cavité mitochondriale.

**<u>II- exemples de synthèses</u>**

**1. Synthèse d'une nitrone amphiphile monocaténaire hydrocarbonée glycosylée**

a. <u>Synthèse du 4-méthyl benzène sulfonate de 2-méthyl-2-nitropropyle **E3**</u>

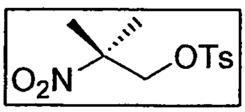

9.6 g de chlorure de tosyle (0.050 mol - 1.2 équiv.) sont dissous dans 30 mL de pyridine. 5 g de 2-méthyl-2-nitropropanol (0.042 mol - 1 équiv.) en solution dans 30 mL de dichlorométhane sont ensuite ajouté goutte à goutte. Le milieu est maintenu à 0°C pendant l'addition puis à température ambiante pendant 48 heures. Le mélange réactionnel est jeté dans 150 mL d'eau glacée sous forte agitation. La phase aqueuse est extraite par 3 fois 50 mL de dichlorométhane. Les phases organiques sont réunies, lavées par 3 fois 75 mL d'HCl 3N puis par 2 fois 75 mL de saumure, séchée sur $Na_2SO_4$ enfin évaporées sous pression réduite. Après recristallisation dans un mélange acétate d'éthyle/cyclohexane, le composé <u>**E3**</u> est obtenu sous forme d'une poudre blanche légère (9.55 g - 0.035mol - 83 %). Ratio front : 0.35 (cyclohexane/acétate d'éthyle 8:2). Pf = 74-75.5°C

RMN $^1H$ (250 MHz, $CDCl_3$) : δ 7.76 (2H, d, J = 8.5 Hz, H arom.), 7.37 (2H, d, J = 8.5 Hz, H arom.), 4.27 (2H, s, $CH_2$-O), 2.46 (3H, s, $CH_3$ du tosyle), 1.56 (6H, s, $CH_3$ du *tert*-butyl)

RMN $^{13}C$ (62.86 MHz, $CDCl_3$) : δ 146.1 ($C^{IV}$ arom.), 132.6 ($C^{IV}$ arom.), 130.7 et 128.6 (CH arom.), 86.3 ($C^{IV}$), 73.2 ($CH_2$-O), 23.5 ($CH_3$ du *tert*-butyl), 22.3 ($CH_3$ du tosyle)

Infra-rouge (KBr, $cm^{-1}$) : $\nu_{(CH\ arom.)}$ = 3059 et 3005, $\nu_{(NO2)}$ = 1543

b. <u>Synthèse du 1-octanesulfanyl-2-methyl-2-nitropropyle **E7a**</u>

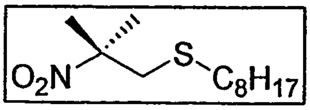

Sous atmosphère d'argon, 4.1 g de *tert*-butylate de potassium (0.039 mol 2 équiv.) sont mis en suspension dans 30 mL de DMF anhydre. Après 20 minutes d'agitation, 6.4 mL d'octanethiol (0.039 mol - 2 équiv.) en solution dans 10 mL de DMF sont additionnés goutte à goutte par l'ampoule à brome. Le milieu prend progressivement un aspect blanc laiteux et après 10 minutes, 5 g de **E3** (0.0183 mol - 1 équiv.) dissous dans 20 mL de DMF sont ajoutés lentement. Le milieu réactionnel est porté à 50°C sous flux d'argon pendant 4 heures. Le mélange est jetté dans 400 mL de saumure glacée puis extrait par 5 fois 50 mL de cyclohexane. La phase organique est lavée par 2 fois 100 mL de saumure, séchée sur $Na_2SO_4$ et évaporée sous pression réduite. Après purification par flash chromatographie sur gel de silice (éluant: cyclohexane/dichlorométhane 9:1 à 8:2), le composé **E7a** (4,4 g - 0.0178 mol - 97 %) est obtenu sous forme d'une huile.
Rf: 0.65 (cyclohexane/acétate d'éthyle 8:2)
RMN $^1$H (250 MHz, $CDCl_3$) $\delta$ 3.04 (2H, s, $C^{IV}$-$CH_2$-S), 2.52 (2H, t, J = 7.25 Hz, $CH_2$-S), 1.64 (6H, s, $CH_3$ du *tert*-butyl), 1.54 (2H, qt, J = 7.25 Hz, $\underline{CH_2}$-$CH_2$-S), 1.40 à 1.20 (10H, m, $CH_2$ de la chaîne), 0.87 (3H, t, J = 7 Hz, $CH_3$ de la chaîne).
RMN $^{13}$C (62.86 MHz, $CDCl_3$) : $\delta$ 87.4 ($C^{IV}$), 41.5 ($C^{IV}$-$\underline{C}H_2$); 33.2 ($CH_2$-S), 30.8, 28.8, 28.1 et 27.7 ($CH_2$ de la chaîne), 24.4 ($CH_3$ du *tert*-butyl), 21.6 ($CH_2$ de la chaîne), 13.1 ($CH_3$ de la chaîne).
Infra-rouge (KBr, $cm^{-1}$) : $\nu_{(NO2)}$ = 1543

c. Synthèse de la *N*-(1,1-Diméthyl-2-octylsulfanyl-ethyl)-hydroxylamine **E7b**

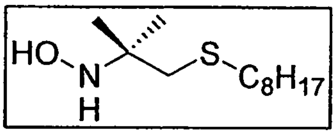

0.247 g de composé nitro **E7a** (1 mmol - 0.25 équiv) est dissous dans 6 mL d'un mélange THF/MeOH (2 : 1) préalablement dégazé à l'argon. Cette solution est ajouté en une fois sur le réactif de Kagan (4 équiv.) sous atmosphère inerte. La réaction, quasi instantanée, est suivit par apparition d'une coloration vert gris (disparition de l'espèce $SmI_2$ au profit de l'espèce $SmI_3$). Après 15 minutes d'agitation, 20 mL d'une solution de $Na_2S_2O_3$ à 10 % est additionné au milieu réactionnel. Le THF est évaporé sous pression réduite. La phase aqueuse est diluée 2 fois puis extraite par 3 fois 30 mL d'acétate d'éthyle la phase organique est lavée par 2 fois 30 mL d'eau distillé puis évaporée sous pression réduite. La purification par chromatographie sur gel de silice (éluant: cyclohexane/acétate d'éthyle 8:2 à 7:3) conduit à l'hydroxylamine **E7b** (164 mg - 0.7 mmol - 70%) sous forme d'une huile translucide.

50 mg du composé **E7a** de départ sont également recueillis et permettent de déterminer un taux de conversion de 88 %.
RMN $^1$H (250 MHz, DMSO) : $\delta$ 7.09 (1H, s, NH), 5.3 (1H, bs, OH), 2.63 (2H, s, $C^{IV}$-$CH_2$-S), 2.55 (2H, t, J = 7.2 Hz, $CH_2$-S), 1.64 (6H, s, $CH_3$ du *tert*-butyl), 1.54 (2H, qt, J = 6.7 Hz et J = 7.2 Hz, $\underline{CH_2}$-$CH_2$-S), 1.40 à 1.20 (10H, m, $CH_2$ de la chaîne), 0.87 (3H, t, J = 7 Hz, $CH_3$ de la chaîne)
RMN $^{13}$C (62.86 MHz, $CDCl_3$) : $\delta$ 57.2 ($C^{IV}$), 40.8 ($C^{IV}$-$\underline{C}H_2$), 33.2 ($CH_2$-S), 31.2, 29.4, 28.6 et 28.5 ($CH_2$ de la chaîne), 23.7 ($CH_3$ du *tert*-butyl), 22.0 ($CH_2$ de la chaîne), 13.9 ($CH_3$ de la chaîne)
Infra-rouge (KBr, $cm^{-1}$) : $\nu_{(NH)}$ = 3246

d. Synthèse de la nitrone hydrocarbonée **A2**

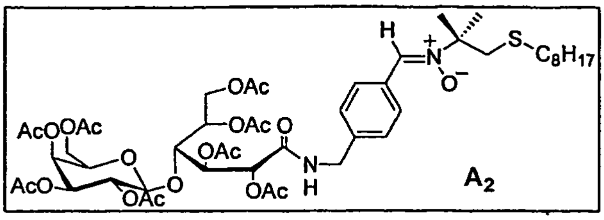

0.5 g d'aldéhyde glycosylé (Ouari O., Chalier F., Pucci B., Tordo P., J. Chem. Soc., Perkin Trans 2 (1998), 2299) (0.615 mmol - 1 équiv.) sont mis en solution sous atmosphère d'argon dans 10 mL de THF anhydre et dégazé. 0.1 g d'hydroxylamine **E7b** (0.430 mmol - 0.7 équiv) en solution dans 2 mL de THF sont additionnées ainsi qu'une pointe de spatule de tamis 4 Å. Le mélange réactionnel est porté à 60 °C sous argon et à l'abri de la lumière. Tous les deux jours, 50 mg d'hydroxylamine (0.215 mmol - 0.35 équiv.) et une pointe de spatule de tamis 4 Å sont additionnés. L'avancement de la réaction est apprécié par CCM et après 8 jours et ajout de 1.75 équiv d'hydroxylamine le milieu réactionnel est filtré sur célite. Après évaporation sous pression réduite des solvants, le brut réactionnel est purifié par chromatographie flash sur gel de silice (éluant :acétate d'éthyle/cyclohexane 7:3). Une purification supplémentaire est effectuée sur résine d'exclusion LH-20 (éluant: Méthanol/Dichlorométhane 1:1) et conduit à la nitrone **A2** (313 mg - 0.304 mmol - 50 %) pure et à 116 mg d'une fraction comportant de l'aldéhyde de départ (dans un rapport 1/3 environ déterminé par RMN $^1$H). Pf= 70°C (dec). $[\alpha]_D$= + 17.8 ° (c, 1, $CH_2Cl_2$).

RMN $^1$H (250 MHz, $CDCl_3$) : δ 8.24 (2H, d, J = 8.1 Hz), 7.49 (1H, s, CH N(O)), 7.25 (2H, d, J = 8.1 Hz), 6.57 (1H, m, NH), 5.67 (1H, d, J = 6.6 Hz, H-2), 5.60 (1H, dd, J = 3.8 Hz et J = 5.8 Hz, H-3), 5.37 (1H, d, J = 3 Hz, H-4'), 5.18 (1H, dd, J = 2.5 Hz et J = 10.3 Hz, H-2'), 5.08 (1H, m, H-5), 4.98 (1H, dd, J = 3.4 Hz et J = 10.4 Hz, H-3'), 4.65 (1H, d, J = 7.9 Hz, H-1'), 4.62 à 4.45 (2H, m, 1 H-6a et H-7a), 4.42 à 4.30 (2H, m, H-4 et H-7b), 4.23 à 3.98 (3H, m, H-6b, H-6'a et H-6'b), 3.90 (1H, t, J = 6.5 Hz), 3.00 (2H, s, $C^{IV}$-$CH_2$-S), 2.41 (2H, t, J = 7.25 Hz, $CH_2$-S), 2.13, 2.05, 2.02, 2.01, 1.98, 1.95 (24H, 6s,$CH_3$-CO), 1.61 (6H, s, $CH_3$ du *tert*-Butyl), 1.43 (2H, m, $\underline{CH_2}$-$CH_2$-S), 1.3 à 1.1 (10H, m, $CH_2$ de la chaîne), 0.82 (3H, t, J = 6.6 Hz, $CH_3$ de la chaîne).

RMN $^{13}$C (62.86 MHz, $CDCl_3$) : δ 170.4, 170.4, 170.0, 170.0, 169.9, 169.7, 169.7, 169.5, 169.2 ($CH_3$-CO), 167.1 (CO-NH), 139.8 ($C^{IV}$ arom.), 131.0 (CH=N(O)), 130.1 ($C^{IV}$ arom.), 129.2, 127.5 (CH arom.), 101.7 (CH-1'), 77.3 (CH-4), 73.4 ($C^{IV}$), 71.7 (CH-2), 70.9 (CH-5' et CH-3'), 69.7 (CH-5), 69.1 (CH-3), 68.9 (CH-2), 66.8 (CH-4'), 61.6, 60.9 ($CH_2$-OAc), 42.9 $CH_2$-NH), 42.4 ($C^{IV}$-$\underline{C}H_2$-S), 33.3 ($CH_2$-S), 31.7, 29.9, 29.0, 29.0, 28.6 ($CH_2$ de la chaîne), 25.7 ($CH_3$ du *tert*-butyl), 22.5 ($CH_2$ de la chaîne), 20.8, 20.7, 20.6, 20.6, 20.6, 20.5, 20.5, 20.4 ($\underline{C}H_3$-CO), 14.0 ($CH_3$ de la chaîne).

MS FAB+ (1027.1 g.mol$^{-1}$) : $[M+H]^+$ = 1027 (5%); $[M+Na]^+$ = 1049 (10%), $[C_{12}H_{25}S]^+$ = 201 (70%).

Après désacétylation des sucres par le méthode de Zemplen on obtient le produit déprotégé :

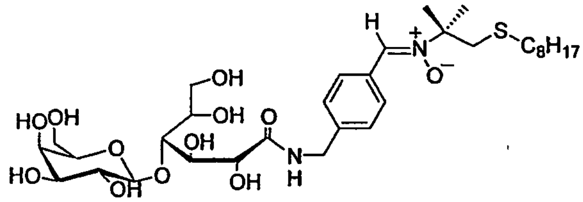

Pf=115°C (dec)

Rf : 0.52 (acétate d'éthyle/méthanol/eau 7:2:1)

$[\alpha]_D$ = +17.2 (0.25c, 1, $CH_3OH$)

RMN $^1$H (250 MHz, $CD_3OD$) : δ 8.28 (2H, d, J = 8.25 Hz), 7.82 (1H, s, CH=N(O)), 7.42 (2H, d, J = 8.25 Hz), 4.65 à 4.35 (4H, m, $CH_2$-NH, H-1', H-2), 4.25 (1H, m, H-3), 4.00 à 3.35 (10H, m, H-4, H-5, $CH_2$-OH, H-4', H-5', H-3' et H-2'), 3.01 (2H, s, $CH_2$-S), 2.43 (2H, t, J = 7.3 Hz, $CH_2$-S), 1.61 (6H, singulet, $CH_3$ du *tert*-butyl), 1.44 (2H, m, $CH_2$), 1.3 à 1.1 (10H, m, $CH_2$), 0.87 (3H, t, J = 6.9 Hz)

RMN $^{13}$C (62.86 MHz, $CD_3OD$) : δ 175.3 (CO-NH), 143.4 ($C^{IV}$ arom.), 136.0 (CH N(O)), 131.1 (CH arom.), 130.6 ($C^{IV}$ arom.), 128.3 (CH arom.), 105.8 (CH-1'), 83.3 (CH-4), 77.2 (CH-5'), 74.8 ($C^{IV}$), 74.6 (CH-3' ou CH-2'), 74.1 (CH-2), 73.2 (CH-5), 72.8 (CH-3' ou CH-2'), 72.5 (CH-3), 70.4 (CH-4'), 63.8, 62.7 ($CH_2$-OH), 43.5, 43.0 ($CH_2$-NH et $CH_2$-S), 34.2 ($CH_2$-S), 32.9, 31.0, 30.3, 30.2, 29.7 ($CH_2$), 26.0 ($CH_3$ du *tert*-butyl), 23.7 ($CH_2$), 14.4 ($CH_3$)

UV (MeOH, mn) : $\lambda_{max}$ = 299

MS FAB+ (690.8 g.mol$^{-1}$): Pas de $[M+H]^+$, $[M+Na]^+$ = 713 (2.5%), $[M+K]^+$ = 729 (1.5%), $[C_{12}H_{25}S]^+$ = 201 (65%)

MS FAB$^-$ (690.8 g.mol$^{-1}$) : $[M-H]^-$ = 689 (très faible)

HPLC (Microsorb C18 - 21.4 mm / 250 mm) : tr =11.4 min

Gradient 70 MeOH - 30 $H_2O$ à 85 MeOH - 15 $H_2O$ de t= 0 à t= 5 min
Isocratique 85 MeOH -15 $H_20$ a partir de t =5 min
Débit 0.6 mL /min

**2. Synthèse de la nitrone fluorocarbonée $A_4$**

a. Synthèse du 1-azido-2-methyl-2-nitropropane

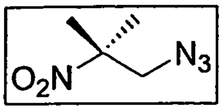

6 g du composé **E3** (0.0218 mol - 1 équiv.) et 2.3 g d'azoture de sodium (0.0353 mol - 1.6 équiv.) sont mis en réaction dans 20 mL de DMF sous activation ultrasonique (grosse sonde - pulse 1 sec/repos 2 sec - amplitude 90 %) en refroidissant le milieu par un bain de glace. Après 3 heures de sonication et disparition complète du produit de départ le milieu réactionnel est repris dans 50 ml de dichlorométhane puis jeté sous vive agitation sur 300 mL de saumure glacée. La phase aqueuse est extraite par 2 fois 50 mL de dichlorométhane puis les phases organiques sont réunies, lavées par 2 fois 75 mL de saumure, séchées sur $Na_2SO_4$ et évaporées sous pression réduite. Après élimination à 50 °C sous vide de la pompe à palette des traces résiduelles de DMF, le composé final (2.66 g - 0.0185 mol - 85 %) est obtenu sous forme d'une huile jaune translucide très liquide.
RMN $^1H$ (250 MHz, $CDCl_3$) : δ 3.74 (2H, s, $CH_2$-$N_3$), 1.60 (6H, s, $CH_3$ du *tert*-butyl)
RMN $^{13}C$ (62.86 MHz, $CDCl_3$) : δ 86.7 ($C^{IV}$), 58.3 ($CH_2$-$N_3$), 23.9 ($CH_3$ du *tert*-butyl)
Infra-rouge (KBr, $cm^{-1}$) : ν $_{(N3)}$ = 2111, ν $_{(NO2)}$ = 1546

b. Synthèse de la 2-methyl-2-nitro-propylamine **E4**

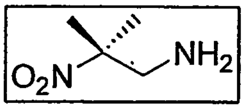

4.08 g d'azoture (0.0283 mol - 1 équiv.) sont mis en solution sous flux d'azote dans 10 mL de THF anhydre et dégazé. 11.3 g de triphénylphosphine (0.0431 mol - 1.50 équiv.) en solution dans 30 mL de THF sont additionnés goutte à goutte à l'azido. Un fort dégagement gazeux se produit. Après 2 heures d'agitation à température ambiante sous atmosphère d'azote, 20 mL d'une solution aqueuse de soude 2N sont ajoutés et le milieu est abandonné 24 heures. Le THF est évaporé sous pression réduite, la phase aqueuse acidifiée jusqu'à pH 1 par addition de 20 mL d'HCl 3N, puis est extraite par 2 fois 30 mL d'acétate d'éthyle. La phase aqueuse est ensuite basifiée par ajout de soude solide jusqu'à pH 10 puis extraite par 3 fois 30 mL de dichlorométhane. La phase organique est lavée par 2 fois 30 mL d'eau, séchée sur $Na_2SO_4$ et évaporée sous pression réduite. L'amine **E4** (2.90 g - 0.0245 mol - 87 %) est obtenue sous forme d'huile jaune.
RMN $^1H$ (250 MHz, $CDCl_3$) : δ 3.07 (2H, s, $CH_2$-$NH_2$),1.57 (6H, s, $CH_3$ du *tert*-butyl)
RMN $^{13}C$ (62.86 MHz, $CDCl_3$) : δ 89.4 ($C^{IV}$), 51.1 ($CH_2$-$NH_2$), 23.6 ( $CH_3$ du *tert*-butyl)

Compte tenu de son instabilité, pour sa caractérisation et sa conservation nous avons synthétisé le chlorhydrate d'ammonium correspondant :

L'amine est reprise dans 60 mL d'éther dans lequel on fait buller de l'HCl gazeux pendant 10 minutes. Le milieu est placé 2 heures à -20°C puis filtré sous pression réduite. Après élimination des traces de solvant à la pompe à palette, le chlorhydrate d'ammonium de **E4** (3.75 g - 0.0243 mol - rendement quantitatif) est obtenu sous forme d'une poudre blanche.
RMN $^1H$ (250 MHz, $D_2O$) δ 3.62 (2H, s, $CH_2$-$NH_3^+Cl^-$), 1.72 (6H, s, $CH_3$ du *tert*-butyl)
RMN $^{13}C$ (62.86 MHz, $D_2O$) : δ 87.0 ($C^{IV}$), 46.9 ($CH_2$-$NH_3$ $^+C^-$), 24.8 ($CH_3$ du *tert*-butyl)
Infra-rouge (KBr, $cm^{-1}$) : ν $_{(NO2)}$ = 1541

c. Synthèse du 4,4,5,5,6,6,7,7,8,8,9,9,9-Tridecafluorononanoyl (2-methyl-2-nitropropyl) amide **E5a**

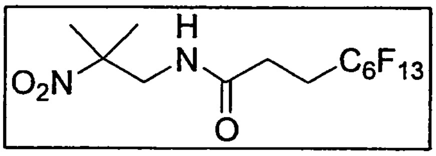

Sous atmosphère d'azote, 2.47 g de DCC (0.0120 mol - 1.2 équiv.) et une pointe de spatule d'HOBt sont mis en solution dans 10 ml de dichlorométhane anhydre. 1.41 g de l'amine **E4** (0.012 mol - 1.2 équiv.) en solution dans 10 mL de dichlorométhane sont ajoutés au milieu. La solution est dégazée pendant quelques minutes, puis 3.86 g d'acide fluoré (0.0098 mol - 1 équiv.) en solution dans 30 mL d'acétate d'éthyle sont additionnés en une seul fois. Après 36 heures d'agitation le milieu réactionnel est filtré, la phase organique est lavée respectivement par 2 fois 50 mL d'HCl 1N, 2 fois 50 mL de saumure puis séchée sur $Na_2SO_4$ et évaporée sous pression réduite. La purification par chromatographie sur gel de silice (éluant: cyclohexane/acétate d'éthyle 8:2 à 7:3) conduit au composé **E5a** (4.4 g - 8.94 mmol - 91%) sous forme d'une poudre blanche. Pf = 87,3-88,8 °C. Rf : 0.37 (cyclohexane/acétate d'éthyle 8: 2).
RMN $^1H$ (250 MHz, $CDCl_3$) : δ 6.11 (1H, massif, NH), 3.76 (2H, d, $C^{IV}$-C$\underline{H}_2$-NH, J = 6.75 Hz), 2.55 à 2.42 (4H, massif, $CH_2$-$CH_2$-Rf), 1.58 (6H, massif, $CH_3$ du *tert*-butyl)
RMN $^{13}C$ (62.86 MHz, $CDCl_3$) : δ 170.5 (CO), 88.7 ($C^{IV}$), 46.1 ($CH_2$ NH-), 24.1 ($CH_3$ du *tert*-butyl)
RMN $^{19}F$ (235 MHz, $CDCl_3$) : δ -81.1 (C$\underline{F}_3$, singulet), -114.8 (C$\underline{F}_2$-$CH_2$, singulet), -122.1, -123.1, et -123.8 (C$\underline{F}_2$, singulet), -126.4 (C$\underline{F}_2$-$CF_3$)
Infra-rouge (KBr, $cm^{-1}$) : $\nu_{(NH)}$ = 3280, ν $(_{C-O)}$ = 1664, $\nu_{(NO2)}$ = 1547, $\nu_{CF2)}$ = 1246

d. 4,4,5,5,6,6,7,7,8,8,9,9,9-Tridecafluorononanoyl (2-hydroxyamino-2-methylpropyl) amide **E5b**

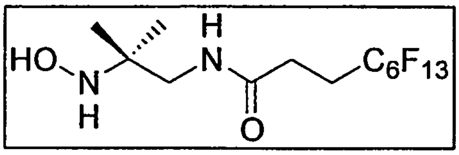

La procédure expérimentale est identique à celle utilisé pour la première hydroxylamine **E7b.** 1.71 g de composé nitro **E7a** (3.5 mmol - 0.25 équiv) en solution dans 21 mL de mélange THF/MeOH, sont additionnés à 140 mL du réactif de Kagan.

Après traitement et purification par chromatographie sur gel de silice (éluant: acétate d'éthyle/méthanol 10:0 à 9.5:0.5), l'hydroxylamine **E7b** (0.82 g - 1.7 mmol - 49 %) est obtenue sous forme d'une poudre blanche.

0.56 g du composé **E7a** de départ sont également recueillis et permettent de déterminer un taux de conversion de 72 %. Pf = 110.5-112.3 °C. Rf: 0.58 (acétate d'éthyle/méthanol 95:5).
RMN $^1H$ (250 MHz, DMSO) : δ 7.79 (1H, t, J = 6.0 Hz, NH), 6.89 (1H, s, NH), 5.23 (1H, bs, OH), 3.05 (2H, d, J = 6.0 Hz, C$\underline{H}_2$-NH), 2.48 (4H, m, $CH_2$-$CH_2$-Rf), 0.86 (6H, s, $CH_3$ du *tert*-butyl)
RMN $^{13}C$ (62.86 MHz, DMSO) : δ 169.7 (CO), 56.9 ($C^{IV}$), 44.7 ($C^{IV}$-$\underline{C}H_2$); 22.4 (2 $CH_3$ du *tert*-butyl)
RMN $^{19}F$ (235 MHz, DMSO) : δ -80.0 ($CF_3$, singulet), -113.4 ($CF_2$-$CH_2$, singulet), -121.5, -122.5 et -123.0 ($CF_2$, singulet), -125.6 (C$\underline{F}_2$-$CF_3$, singulet)

e. Synthèse de la nitrone fluorocarbonée **A4**

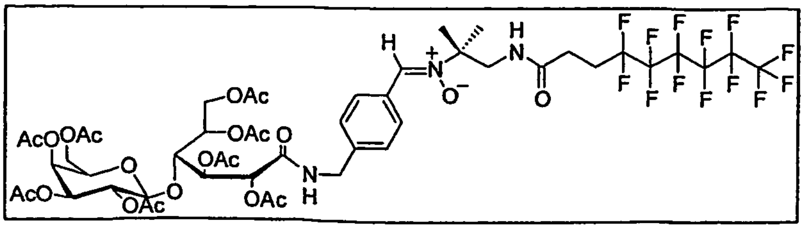

La procédure expérimentale est identique à celle utilisé pour la première nitrone.

0.61 g d'aldéhyde (0.75 mmol - 1 équiv.) sont mis en réaction avec l'hydroxylamine **E7b** (0.26g - 0.7 équiv.) dans 15 mL de THF. Après 10 jours d'agitation et addition de 0.35 g supplémentaire d'hydroxylamine (0.732 mmol - 0.98 équiv.) la réaction est stoppée.

Les purifications sont effectuées par chromatographie flash sur gel de silice (éluant : Acétate d'éthyle/Méthanol 10:0 à 95:5) et par chromatographie d'exclusion sur résine LH-20 (éluant :Méthanol/Dichlorométhane 1:1). La nitrone $\mathbf{A_4}$ est obtenue pure (0.564 g - 0.443 mmol - 60 %) sous forme d'une mousse blanche. L'aldéhyde de départ (115 mg - 0.142 mmol) est également récupéré ce qui permet de déterminer un taux de conversion de 73 %. Pf = 95 °C (dec)
RMN $^1$H (250 MHz, $CDCl_3$) : δ 8.21 (2H, d, J = 8.1 Hz), 7.49 (1H, s, CH=N(O)), 7.31 (2H, d, J = 8.1 Hz), 6.95 (1H, t, J = 6 Hz, NH, 6.76 ( 1H,t, J = 6 Hz, NH), 5.45 à 3.80 (15H), 3.69 (2H, d, J = 6.0 Hz, $C^{IV}$-$CH_2$-NH), 2.70 à 2.35 (4H, m, $CH_2$-$CH_2$-Rf), 2.17, 2.16, 2.08, 2.07, 2.06, 2.05, 2.04, 1.98 (24H, 8s, $CH_3$-CO), 1.60 (6H, s, $CH_3$ du *tert*-Butyl)
RMN $^{13}$C (62.86 MHz, $CDCl_3$) : δ 170.6 (CO-NH + $CH_3$-$\underline{C}$O), 170.4, 170.2, 170.1, 170.0 169.8, 169.7, 169.3 ($CH_3$-CO), 167.3 (CO-NH), 140.6 ($C^{IV}$ arom.), 131.5 (CH-N(O)), 129.8 ($C^{IV}$ arom.), 129.4, 127.7 (CH arom.), 101.9 (CH-1'), 77.5 (CH-4), 73.4 ($C^{IV}$), 71.7 (CH-2), 71.0 (CH-5' et CH-3'), 70.0 (CH-5), 69.3 (CH-3), 69.1 (CH-2), 66.9 (CH-4'), 61.8, 60.9 ($CH_2$-OAc), 47.3, 43.1 ($CH_2$-NH), 27.0 0, 24.9 ($CH_3$ du *tert*-butyl), 20.9, 20.8, 20.7, 20.7, 20.6, 20.5 ($CH_3$-CO)
RMN $^{19}$F (235 MHz, $CDCl_3$) : δ -81.1 ($CF_3$, s), -115.0 ($CF_2$-$CH_2$, s), - 122.3,-123.2, -123.9 ($CF_2$, s), -126.5 ($\underline{CF_2}$-$CF_3$, s)
MS FAB$^+$ (1272.0 g.mol$^{-1}$) : [M+H] = 1273 (1.5%), [M+Na] = 1295 (3.5%)

Après désacétylation des sucres par la méthode de Zemplen on obtient le produit déprotégé :

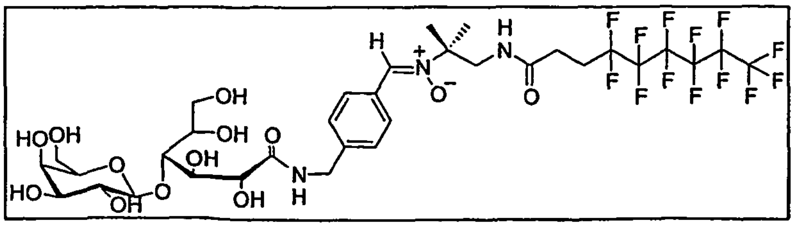

Pf =150 °C (dec)
$[\alpha]_D$ = +14.4 (0.25c, 1, $CH_3OH$)
UV (MeOH, nm) : $\lambda_{max}$= 299
Rf : 0.47(acétate d'éthyle /méthanol/eau 7:2:1)
RMN $^1$H (250 MHz, $CD_3OD$) δ 8.33 (2H, d, J = 8.4 Hz), 7.86 (1H, s, CH=N(O)), 7.47 (2H, d, J = 8.5 Hz), 4.65 à 4.45 (4H, m, $CH_2$-NH, H-1', H-2), 4.3 (1H, m, H-3), 4.05 à 3.87 (2H, m, H-4 et H-5),3.87 à 3.66 (7H, m, $CH_2$-OH, H-4' et $CH_2$-NH), 3.66 à 3.45 (3H, m, H-5', H-3' et H-2'), 2.55 à 2.40 (4H, m, $CH_2$-$CH_2$-Rf), 1.59 (6H, singulet, $CH_3$ du *tert*-butyl)
RMN $^{13}$C (62.86 MHz, $CD_3OD$) : δ 174.0, 171.9 (CO-NH), 142.1 ($C^{IV}$ arom.), 134.6 (CH=N(O)), 129.7 (CH arom.), 129.2 ($C^{IV}$ arom.), 126.9 (CH arom.), 104.4 (CH-1'), 82.0 (CH-4), 75.8 (CH-5'), 73.5 ($C^{IV}$), 73.4 (CH-3' ou CH-2'), 72.7 (CH-2), 71.8 (CH-5), 71.4 (CH-3' ou CH-2'), 71.2 (CH-3), 69.0 (CH-4'), 62.4 ($CH_2$-6) , 61.3 ($CH_2$-6'), 46.3 ($C^{IV}$-$CH_2$-NH), 42.1 ($C^{IV}$arom.-$\underline{C}H_2$-NH), 26.0 ($CH_2$-$CH_2$-Rf), 23.3 ($CH_3$ du *tert*-butyl)
RMN $^{19}$F (235 MHz, $CD_3OD$) : δ -82.1 ($CF_3$, s), -115.3 ($CF_2$-$CH_2$, s), - 122.6,-123.6, -124.3 ($CF_2$, s), -127.0 ($\underline{CF_2}$-$CF_3$, s)
MS FAB$^+$ (935.7 g.mol$^{-1}$) : $[C_{13}H_{13}F_{13}NO]^+$ = 446 1%), $[C_9H_4F_{13}O]^+$ = 375 (8%)
MS FAB$^-$(35.7 g.mol$^{-1}$) : [M-H] = 934 (très faible)
Colonne préparative ( Microsorb C18 - 21.4 mm / 250 mm) : tr = 9.800
Gradient 70 MeOH - 30 $H_2O$ à 80 MeOH-20 $H_2O$ de t = 0 à t = 5 min
Gradient 80 MeOH - 20 $H_2O$ à 82 MeOH -18 $H_2O$ de t = 5 à t = 8 min
Isocratique 82 MeOH -18 $H_2O$ a partir de t = 8 min
Débit 0.8 mL /min

**3. Synthèse de la nitrone hydrocarbonée ionique $B_1$**

a. Synthèse de l'iodure de [4-(1,3-Dioxolanl-2-yl-benzyl) trimethyl ammonium

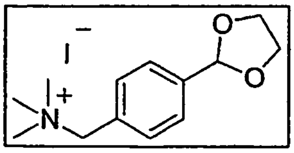

1.25 g d'amine **E15** (7 mmol - 1 équiv.) sont dissous dans 4 mL de DMF dans un tube scellé. 2.58 g de tributylamine (14 mmol - 2 équiv) sont ensuite ajoutés en une fois sous agitation. Le milieu est refroidi à 0 °C et 5.2 g d'iodure de méthyle (35mmol - 5 équiv) sont additionnés lentement au milieu. Le tube scellé est fermé et l'agitation est poursuivie à température ambiante pendant 20 heures. Le brut réactionnel est repris dans l'AcOEt et le précipité obtenu est filtré, repris dans l'éther puis à nouveau filtré. L'ammonium (1.7 g - 4.9 mmol - 70 %) est obtenu sous forme d'une poudre blanche.
RMN $^1$H (250 MHz, DMSO-d6) : δ 7.59 (4H, s, Harom.), 5.80 (H, s, H de l'acétal), 4.61 (2H, s, $CH_2$-NH), 4.2 à 3.9 (4H, AA'BB', $CH_2$-O), 3.06 (9H, s, $CH_3$-N)
RMN $^{13}$C (62.86 MHz, DMSO-d6) : δ 140.6 ($C^{IV}$ arom.), 133.3 (CH arom) 129.6 ($C^{IV}$ arom.), 127.5 (CH arom.), 102.7 (CH acétal), 67.6 ($CH_2$-N), 65.4 ($CH_2$-O), 52.2 ($CH_3$-N)
Analyse centésimale (C13H20NO2I, 0.83 H2O) calculé C 41.69, H 4.60, N 4.42 trouvé C 41.69, H 4.58, N 4.31.

b. Synthèse de l'iodure de (4-Formyl-benzyl)-triméthyl-ammonium **E26**

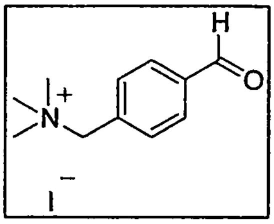

0.38 g du dioxolane ammonium (1.08 mmol - 1 équiv.) sont dissous dans 10 mL de mélange acide acétique/eau 1:1. Après 12 heures d'agitation le milieu réactionnel est évaporé sous vide et les traces de solvant sont éliminées à la pompe à palette. Le composé **E26** (0.34 g - 1.08 mmol - rdt quantitatif) est obtenu sous forme d'une poudre brune foncée.
RMN $^1$H (250 MHz, DMSO-d6) : δ 10.12 (1H, s, CHO), 8.06 (2H, d, J = . 8 Hz, H arom.), 7.80 (2H, d, J = 8 Hz, H arom.), 4.69 (2H, s, $CH_2$-NH), 3.09 (9H, s, $CH_3$-N)
RMN $^{13}$C (62.86 MHz, DMSO-d6) : δ 193.4 (CHO), 137.6, 134.8 ($C^{IV}$ Arom), 134.1, 130.2 (CH Arom), 62.2 ($CH_2$N), 52.6 ($CH_3$N)

c. Synthèse de la nitrone ionique fluorocarbonée **B2**

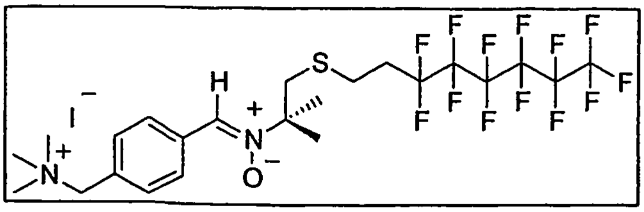

0.25 g de composé **E26** (0.82 mmol - 1 équiv.) et 0.48 g d'hydroxylamine **E7b** (1.02 mmol - 1.25 équiv.) sont mis en solution dans 5 mL de pyridine dégazé à l'argon. Le milieu réactionnel est porté à 80°C a l'obscurité sous atmosphère d'argon pendant 42 heures. Le mélange réactionnel est ensuite évaporée sous pression réduite et les traces de pyridine sont éliminées à la pompe à palette. La nitrone est obtenue sous forme de poudre blanche (0.35 g - 0.47 mmol - 57 %) après deux cristallisations successives dans un mélange MeOH/éther. Pf= 171-173

RMN $^1H$ (250 MHz, $CD_3OD$) : δ 8.54 (2H, d, J = 8.4 Hz, Harom.), 8.03 (1H, s, CH=N(O)), 7.71 (2H, d, J = 8.4 Hz, Harom.), 4.65 (2H, s, $CH_2$-N), 3.18 (11H, s, $CH_3$-N + $C^{IV}$-CH2-S), 2.7 (2H, m, $CH_2$-S), 2.45 (2H, $CH_2$-$C\underline{H}_2$-Rf), 1.71 (6H, s, $CH_3$ du *tert*-Butyl)

RMN $^{13}C$ (62.86 . MHz, $CD_3OD$) : δ 133.3 ($C^{IV}$ arom.), 132.8 (CH=N(O)), 132.7 (CH arom.), 129.8 ($C^{IV}$ arom.), 129.7 (CH arom.), 73.9 ($C^{IV}$), 68.5 ($CH_2$-N), 51.9, 51.9, 51.8 ($CH_3$-N), 41.3 ($CH_2$-S), 31.9 ($CH_2$-$\underline{C}H_2$-Rf), 24.6 (CH3 *tert*-butyl), 23.0 ($\underline{C}H_2$-$CH_2$-Rf)

RMN $^{19}F$ (235 MHz, $CD_3OD$) : δ -82.3 ($CF_3$, singulet), -115.2 ($CF_2$-$CH_2$, singulet), -112.9, -123.9, -124.3 ($CF_2$, singulet), -127.3 ($C\underline{F}_2$-$CF_3$, singulet)

UV (MeOH, nm) : $\lambda_{max}$ = 304 nm

HR MS FAB$^+$ (754.4 g.mol$^{-1}$) : m/z théorique: 755.0838 pour $C_{23}H_{29}F_{13}IN_2OS$ ( $[M+H]^+$)

m/z observé: 755.0851

MS FAB$^+$ (754.4 g.mol$^{-1}$) : $[2M+H]^+$ = 1510, $[2M-I]^+$ = 1381 (5%), $[M+H]^+$ = 755 (2.5%), $[M-I]^+$ = 627 (100%), $[C_{12}H_{12}F_{13}S]^+$ = 435 (100%)

**4. Synthèse de la nitrone bicaténaire hydrocarbonée C1**

<u>a. Synthèse de 1' Heptadecyl-carbamic acid 3-heptadecylcarbamoyloxy-2-methyl-2-nitro-propyl ester **E9a**</u>

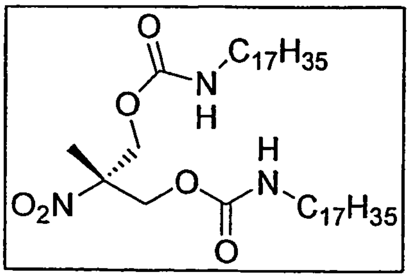

6.31 g d'acide stéarique (0.022 mol - 3 équiv) sont mis en suspension dans 50 mL de toluène anhydre sous atmosphère d'argon. 2.47 g de triéthylamine (0.024 mol - 3.3 équiv) et 6.71 g de diphenylphospsorylazide (0.024 mol - 3.3 équiv.) sont additionnés et le milieu est porté à 60 °C. Après 2 heures d'agitation, 1 g de 2-nitro-2-méthyl-1,3-propanediol (0.0074 mol - 1 équiv.) et une pointe de spatule de DABCO sont mis en suspension et l'agitation est poursuivie pendant 12 heures. Le brut réactionnel est dilué avec 100 mL d'acétate d'éthyle, lavé par 3 fois 50 ml d'HCl 1N, 3 fois 50 mL de $NaHCO_3$ saturée et enfin lavé par 2 fois 50 mL de saumure. La phase organique est séchée sur $Na_2SO_4$ et évaporée sous pression réduite. Après 3 cristallisations successives le composé **E9a** (2.02 g -2.89 mmol - 40 %) est obtenu sous forme d'une poudre blanche. Pf : 75-76.2 °C

Rf : 0.42 (cyclohexane/acétate d'éthyle 8:2)

RMN $^1H$ (250 MHz, $CDCl_3$) : δ 4.77 (2H, m, NH), 4.45 (2H, système AB,$CH_2$-O), 3.15 (2H, q, J=9.8 Hz, $C\underline{H}_2$-NH), 1.59 (3H, s, $CH_3$ du *tert-butyl),* 1.47 (2H, m, $C\underline{H}_2$-$CH_2$-NH), 1.24 (55H, m, $CH_2$ de la chaîne), 0.87 (3H, t, $CH_3$ de la chaîne)

RMN $^{13}C$ (62.86 MHz, $CDCl_3$) : δ 155.1 (CO), 88.1 ($C^{IV}$), 65.1 ($CH_2$-O), 41.3 ($CH_2$-NH), 31.9, 29.8, 29.7, 29.6, 29.5, 29.4, 29.3 ($CH_2$ de la chaîne), 26.7 ($\underline{C}H_3$ du *tert-butyl),* 22.7, 18.5 ($CH_2$ de la chaîne), 14.1 ($CH_3$ de la chaîne)

Infra-rouge (KBr, $cm^{-1}$) : $\nu_{(NH)}$ = 3392 , $\nu_{(CO)}$ = 1720 et 1703 , $\nu_{(NO2)}$ = 1549

b. Synthèse de 1' heptadecylcarbamoyl 3-heptadecylcarbamoyloxy-2-hydroxylamino-2-methyl-propyl ester **E9b**

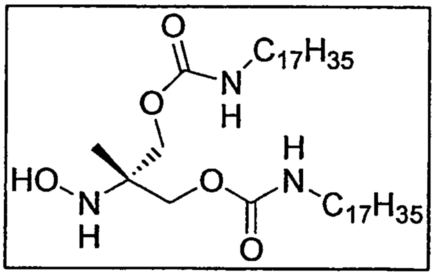

La procédure expérimentale est identique à celle utilisée pour synthétiser les composés **E5b** et **E7b.**

1.39 g de composé nitro **E9a** (2.0 mmol - 0.25 équiv) en solution dans 20 mL de mélange THF/MeOH, sont additionnés à 80 mL du réactif de Kagan.

Après traitement et purification par chromatographie sur gel de silice (éluant: dichlorométhane/acétate d'éthyle 10:0 à 5:5), l'hydroxylamine **E9b** (0.8 g - 1.17 mmol- 60%) est obtenue sous forme d'une poudre blanche.

0.24 g du composé **E9a** de départ sont également recueillis et permettent d'obtenir un taux de conversion de 71 %. Pf = 80-81.6 °C

Rf: 0.51 (cyclohexane / acétate d'éthyle 5 : 5)

RMN $^1H$ (250 MHz, $CDCl_3$) : δ 4.83 (2H, t, J = NH), 4.45 (4H, système AB,$CH_2$-O),3.17 (4H, q, J = 9.8 Hz, $C\underline{H}_2$-NH), 1.49 (2H, m, $C\underline{H}_2$-$CH_2$-NH), 1.25 (55H, m, $CH_2$ de la chaîne), 1.07 (3H, s, $CH_3$ du *tert*-butyl), 0.88 (3H, t, $CH_3$ de la chaîne)

RMN $^{13}C$ (62.86 MHz, $CDCl_3$) : δ 156.7 (CO), 88.1 ($C^{IV}$), 64.7 ($CH_2$-O), 41.2 ($CH_2$-NH), 31.9, 29.8, 29.7, 29.6, 29.5, 29.4, 29.3 ($CH_2$ de la chaîne), 26.8 ($\underline{C}H_3$ du *tert*-butyl), 22.7, 16.8 ($CH_2$ de la chaîne), 14.1 ($CH_3$ de la chaîne)

c. Synthèse de la nitrone biantennée hydrocarbonée **C1**

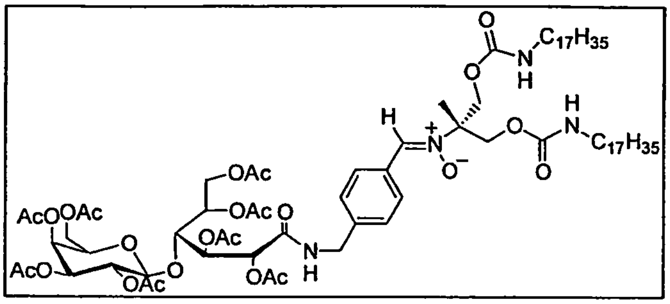

0.5 g d'aldéhyde **E20** (0.616 mmol - 1 équiv.) sont mis en solution en présence de tamis moléculaire 4 Å et de 0.3 g d'hydroxylamine **E9b** (0.44 mmol - 0.71 équiv.) dans 6 mL de THF anhydre et dégazé. 1.2 mL d'acide acétique glacial est additionné et le milieu est chauffé à 50 °C sous argon à l'abri de la lumière.

Après 48 et 96 heures 0.2 g d'hydroxylamine (0.29 mmol - 0.47 équiv) sont rajoutés et le milieu réactionnel est filtré sur célite au bout de 5 jours de réaction.

Les purifications par chromatographie flash sur gel de silice (éluant :Acétate d'éthyle/dichlorométhane 7:3 à 8:2) et par chromatographie d'exclusion sur résine Séphadex LH-20 (éluant :Ethanol/Dichlorométhane 1:1) permettent d'obtenir la nitrone de type C (0.58 g - 0.392 mmol-63 %), quasi exempte de traces d'aldéhyde, sous forme de poudre blanche. 80 mg d'aldéhyde pur sont également récupérés permettant de déterminer un taux de conversion de 76 %. $[\alpha]_D$ = + 16.9° (c, 1, $CHCl_3$) à 20 °C.

Rf : 0.37 (Acétate d'éthyle/Dichlorométhane 8:2)

RMN $^1H$ (250 MHz, $CDCl_3$) : δ 8.28 (2H, d, J = 8.1 Hz, H arom.), 7.45 (1H, s, CH=N(O)), 7.31 (2H, d, J = 8.5 Hz, H

arom.), 6.65 (1H, t, J = 5.8 Hz, NH amide), 5.75 à 5.55 (2H, m, H-2 et H-3), 5.35 (1H, d, J = 3 Hz), 5.25 à 4.80 (5H, m, H-2', H-5, H-3' et NH uréthane), 4.70 à 4.25 (9H,m, H-1', H-6a et H-7a, H-4, H-7b et $CH_2$-O-CO-NH), 4.20 à 3.80 (4H, m, H-6b, H-6'a, H-6'b et H-5'), 3.14 (4H, dd, J = 6.7 Hz, $CH_2$-NH-CO-O), 2.16, 2.15, 2.09, 2.05, 2.04, 1.98, 1.92 (24H, 8s, $CH_3$-CO), 1.60 (3H, s, $CH_3$ du *tert* butyl), 1.55 à 1.10 (60H, m, $CH_2$ de la chaîne), 0.87 (6H, t, J = 6.4 Hz, $CH_3$ de la chaîne)

RMN $^{13}C$ (250 MHz, $CDCl_3$) : δ 170.6, 170.3, 170.2, 170.0, 169.9, 169.8, 169.4, ($CH_3$-$\underline{C}$O), 167.3 (CO-NH), 155.7 (O-CO-NH), 140.3 ($C^{IV}$arom.), 132.4 (CH=N(O)), 130.0 ($C^{IV}$ ou CH arom.), 129.6 ($C^{IV}$ ou CH arom.),127.8 (CH arom.), 101;9 CH-1') 77.4 (CH-4), 75.1 ($C^{IV}$), 71.7 (CH-2), 71.1 (CH-5' et CH-3'), 69.9 (CH-5), 69.3 (CH-3), 69.1 (CH-2), 66.9 (CH-4'), 65.5 ($CH_2$-O-CO-NH), 61.8 et 61.0 ($CH_2$-OAc), 43.2 ($CH_2$-NH), 41.3 ($CH_2$-NH-CO-O), 32.0, 29.9, 29.8, 29.7, 29.7, 29.6, 29.4, 29.3 ($CH_2$ de la chaîne), 26.8 (CH3 du *tert*-Butyl), 22.8 ($CH_2$ de la chaîne), 20.9, 20.9, 20.8, 20.7, 20.7, 20.6 ($CH_3$-CO), 14.2 ($CH_3$ bout de chaîne)

MS FAB$^+$ (1477.8 g.mol$^{-1}$) : [M+H] = 1478 (16 %), [M+Na] =1500 (6 %).

Après désacétylation des sucres par la méthode de Zemplen on obtient le produit déprotégé :

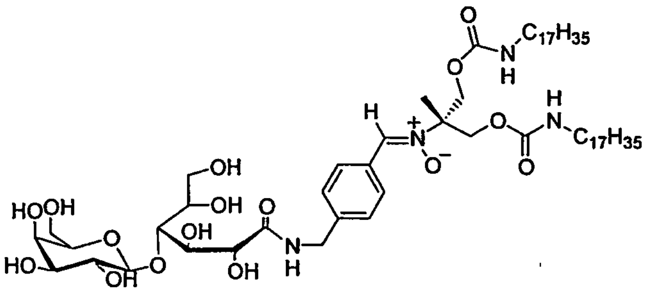

La purification de la nitrone **$C_1$** est réalisée flash par chromatographie sur gel de silice (éluant : chloroforme/méthanol/eau 8:2:0.1) puis par chromatographie d'exclusion de taille sur Sephadex LH-20 (éluant : dichlorométhane/méthanol 7:3).

$[\alpha]_D$ = +7.6° (0.25c, 1, $CHCl_3$)

Rf : 0.28 (chloroforme/méthanol/eau 8:2:0.1)

Pf=190°C(déc)

RMN $^1H$ (250 MHz, DMSO-d6) : δ 8.28 (2H, d, J = 8.2 Hz, H arom.), 8.07 (1H, t, J = 6.3 Hz, NH amide), 7.72 (1H, s, CH N(O)), 7.35 (2H, d, J = 8.3 Hz, H arom.), 7.08 (2H, m, NH uréthane), 4.60 à 4.00 (9H, m, $CH_2$-NH, $CH_2$-O-CO-NH, H-1', H-2 et H-3), 3.78 (2H, m, H-4 et H-5), 3.70 à 3.40 (8H, m, $CH_2$-OH, H-2', H-3', H-4' et H-5'), 2.94 (4H, m, $CH_2$-NH-CO-O), 1.54 (3H, s, $CH_3$ du *tert-butyl),* 1.45 à 1.10 (60H, m, $CH_2$), 0.87 (6H, t, J = 6.6 Hz, $CH_3$)

RMN $^{13}C$ (62.86 MHz, DMSO-d6) : δ 173.0 (CO-NH), 156.1 (O-CO-NH), 142.3 ($C^{IV}$ arom.), 132.1 (CH=N(O)), 130.0 ($C^{IV}$ arom.), 129.1 (CH arom.), 127.2 (CH arom.), 105.1 (CH-1'), 83.4 (CH-4), 76.2 (CH-5'), 74.9 ($C^{IV}$), 73.7 (CH-3' ou CH-2'), 72.6 (CH-2), 71.9 (CH-5), 71.6 (CH-3' ou CH-2'), 71.1 (CH-3), 68.7 (CH-4'), 65.1 ($CH_2$-O-CO-NH), 62.8, 61.1 ($CH_2$-6 et $CH_2$-6'), 42.2 ($CH_2$-NH), 40.7 ($CH_2$-NH-CO-O), 31.8, 29.8, 29.6, 29.2 ($CH_2$ de la chaîne), 26.7 ($CH_3$ du *tert*-butyl), 22.6 ($CH_2$ de la chaîne), 14.4 ($CH_3$ de la chaîne)

MS FAB$^+$ (1140.77 g.mol$^{-1}$) : [M + Na] = 1164, [M + H] =1142

**III-Mesure de l'hydrophobie des molécules de l'invention :**

L'un des objectifs de l'invention est, de moduler la HLB de pièges à radicaux libres afin de favoriser le passage transmembranaire et le transport *in vivo.*

Dans cette optique il était important de déterminer le coefficient de partition P de ces composés.

En effet, Hansch et ses collaborateurs (Hansch, C.; Steward, A. R.; Anderson, S. M.; Bentley, D. *J. Med. Chem.* **11,** 1 (1968)) ont pu établir au cours d'une étude sur l'efficacité d'action de différents hypnotiques en fonction de leur hydrophobie la relation suivante:

$$\log 1/C = -k(\log P)^2 + k'(\log P) + k''$$

*C* : la concentration molaire produisant une réponse biologique standard

*k, k'* et *k"* : des constantes déterminée par la méthode des moindres carrés.

Ainsi plus un composé sera hydrophobe, plus la valeur log *P* sera supérieur à 0 et plus les interactions avec

la phase lipidique seront élevées.

Nous avons déterminé le coefficient de partition de ces nitrones par Chromatographie Liquide Haute Performance en Phase Inverse. (Lambert, W. J. J. Chromtogr. 656, 469 (1993) ; Dorsey, J. G.; Kahaledi, M. G. J. Chromtogr. 656, 485 (1993)).

Thomas a également utilisé cette approche chromatographique pour déterminer l'hydrophobie de spin traps cycliques dérivés de la PBN (Fevig, T. L.; Bowen, S. M.; Janowick, D. A.; Jones, B. K.; Munson, H. R.; Ohlweiler, D. F.; Thomas, C. E. J. Med Chem. 39, 4988 (1996)) L'estimation du coefficient de partition Octanol/eau par HPLC en phase inverse ($K_{OW}$) est hautement dépendante du temps de rétention des composés et par la suite du coefficient de capacité *k'*. Il peut être exprimé par la relation suivante:

$$\log K_{OW} = a \log k' + b$$

Dans laquelle *a* et *b* sont des constantes empiriques qui caractérisent le système de solvant.

Expérimentalement *k'* est déterminé par la formule suivante pour différents mélanges éluant méthanol/eau.

$$k' = (t_R - t_0)/t_0$$

dans laquelle $t_R$ représente le temps de rétention de l'échantillon et $t_0$ le temps d'élution de la phase mobile

Il convient ensuite d'extrapoler par régression linéaire la valeur de *k'* pour une phase composée par 100 % d'eau afin d'obtenir la valeur $k_W$.

Nous avons procédé de cette manière pour les composés dérivés de l'acide lactobionique de type **A**. Nous avons également par souci de comparaison et de validation de notre modèle, déterminé l'hydrophobie de la PBN et de la TA1PBN.

Le tableau 3 résume les valeurs de temps de rétention moyen obtenu à partir d'un minimum de 3 valeurs établies au minimum sur 2 jours différents.

La régression linéaire des valeurs de *k'* obtenue suivant les phases mobiles utilisées permet d'obtenir une équation de droite du type :

$$y = ax + b$$

dans laquelle y représente log *k'* et a représente log $k'_W$
x représente la fraction en méthanol de l'éluant.

Tableau 3: Détermination des valeurs de log *k'* par HPLC

| Phase Mobile (MeOH/$H_2O$) | **60/40** | | **70/30** | | **80/20** | | **90/10** | |
|---|---|---|---|---|---|---|---|---|
| **Nitrones** (C en mg/ml) | 225-235 bars | | 200-220 bars | | 175-195 bars | | 140-160 bars | |
| | **$t_R$** | **log *k'*** | **$t_R$** | **log *k'*** | **$t_R$** | **log *k'*** | **$t_R$** | **log *k'*** |
| MEOH | 3.98 | | 3.97 | | 3.94 | | 3.89 | |
| PBN (0.64 mg/ml) | 7.98 | 0.0019 | 6.09 | -0.2711 | 5.07 | -0.5405 | 4.47 | -0.8203 |
| **TA1PBN** (0.26 mg/ml) | - | - | 28.92 | 0.7985 | 8.46 | 0.0593 | 4.85 | -0.6057 |
| **A2** (0.52 mg/ml) | - | - | 22.43 | 0.6676 | 9.06 | 0.1140 | 5.31 | -0.4344 |
| **A3** (0.52 mg/ml) | - | - | 29.28 | 0.8047 | 9.04 | 0.1119 | 4.87 | -0.5967 |
| A1 (0.48 mg/ml) | 9.98 | 0.1776 | 6.14 | -0.2624 | 4.76 | -0.6840 | - | - |
| **A4** (0.57 mg/ml) | 41.41 | 0.9731 | 12.64 | 0.3394 | 6.12 | -0.2562 | - | - |
| **A5** (0.52 mg/ml) | 8.21 | 0.0264 | 5.51 | -0.4143 | 4.49 | -0.8524 | - | - |

Nous avons procédé de la même manière pour les 4 autres dérivés et les résultats sont résumés dans tableau suivant (tableau 4).

Tableau 4 : Détermination des valeurs de log *k*'w par HPLC

| | a | $R^2$ | log $k'_w$ |
|---|---|---|---|
| **PBN** | -2.7366 | 0.9999 | 1.6447 |
| **TA1PBN** | -7.0209 | 0.9991 | 5.7008 |
| $\mathbf{A_2}$ | -5.5098 | 1 | 4.5235 |
| $\mathbf{A_3}$ | -7.0079 | 1 | 5.7129 |
| $\mathbf{A_1}$ | -4.3082 | 0.9998 | 2.7595 |
| $\mathbf{A_4}$ | -6.1468 | 0.9997 | 4.6549 |
| $\mathbf{A_5}$ | -4.3942 | 1 | 2.6635 |

Dans uni souci de clarté nous avons reporté les valeurs de log $k'_w$ des différentes nitrones sur un histogramme (Figure 11).

Les résultats obtenus nous permettent d'effectuer plusieurs remarques et conclusions:

1- Pour les composés à chaînes hydrocarbonées, les valeurs obtenues sont en accord avec nos attentes. Plus la chaîne comporte d'atomes de carbones plus l'hydrophobie est élevée. D'autre part la nature des jonctions de chaîne joue un rôle non négligeable dans la valeur du log *k'*, une liaison amide ou uréthane étant par nature plus polaire qu'une jonction thioéther. On obtient donc en terme d'hydrophobie le classement croissant suivant :

$$A_5 < A_1 < A_2$$

2- Pour les composé fluorés, le composé $\mathbf{A_3}$ présente une plus grande affinité pour les milieux lipidiques que le composé $\mathbf{A_4}$ ce qui semble en accord avec leur valeurs de CMC respectives. Cependant, les composés $\mathbf{A_3}$ et $\mathbf{A_4}$ possèdent des valeur de log $k'_w$ proches alors que leurs valeurs de CMC varient de plus du double. Ceci provient de la nature des chaînes fluorés qui présentent des propriétés tensioactives particulières. Il convient donc de bien séparer notion de tensioactivité et notion d'hydrophobie. On obtient donc en terme d'hydrophobie le classement croissant suivant :

$$A_5 < A_1 < A_2 < A_4 < A_3$$

3- la valeur de log $k'_w$ de la PBN est sensiblement plus faible que l'ensémble des composés synthétisés. Selon la théorie de Hansch, nous pouvons en déduire une meilleure pénétration trans membranaire des composés et donc une meilleure activité de piège à radicaux libres.

4- La valeur de log $k'_w$ de la TA1PBN est sensiblement identique à celle du composé $\mathbf{A_3}$.

## Revendications

1. Composé, **caractérisé en qu'**il répond à la formule (I) :

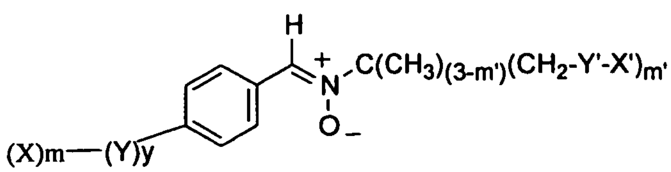

(I)

dans laquelle :

X représente un groupement hydrophile choisi parmi un mono ou un polysaccharide, ainsi que les dérivés aminés de mono et de polysaccharides, une chaîne polyoxyde d'éthylène, une chaîne peptidique, un groupement polaire ionique choisi parmi un ammonium quaternaire, un oxyde d'amine, un groupement carnitine ;
m représente un entier égal à 1, 2 ou 3 ;
Y représente un bras espaceur destiné à relier le noyau aromatique et les substituants hydrophiles X ;
Y est choisi parmi les fonctions ester, amide, urée, uréthane, éther, thio-éther, amine, les chaînes hydrocarbonées en $C_1$-$C_6$, éventuellement interrompues par une ou plusieurs fonctions ester, amide, urée, uréthane et par un ou plusieurs ponts éther, amine ou thio-éther ;
y représente un entier égal à 0 ou à 1 ;
Y' représente un groupement choisi parmi une fonction ester, une fonction amide, une fonction urée, une fonction uréthane, un pont éther, un pont thio-éther ;
m' est un entier choisi parmi 1 et 2 ;
X' représente une chaîne alkyle en $C_4$-$C_{14}$ éventuellement substituée par un ou plusieurs atomes de fluor.

2. Composé selon la revendication 1, **caractérisé en ce que** X représente un groupement choisi parmi : le glucose, le lactose, le fructose, le mannose, le galactose, le ribose, le maltose, la glucosamine, le saccharose et le lactobionamide.

3. Composé selon la revendication 1, **caractérisé en ce que** X représente un groupement choisi parmi les chaînes polyoxyde d'éthylène comprenant de 30 à 100 unités oxyde d'éthylène, de préférence de 50 à 60 unités.

4. Composé selon la revendication 1, **caractérisé en ce que** X représente un groupement choisi parmi

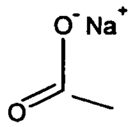

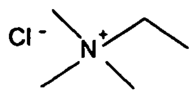

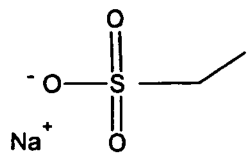

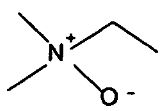

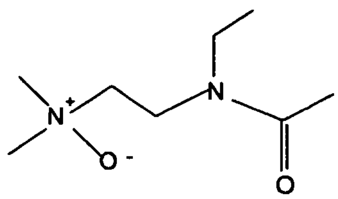

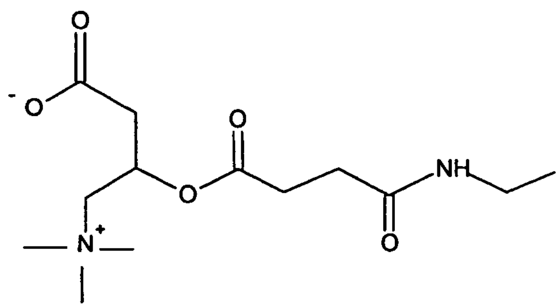

5. Composé selon la revendication 1, **caractérisé en ce que** l'une ou moins des conditions ci-dessous est satisfaite :

X représente un groupement choisi parmi : la lactobionamide, la carnitine ou une chaîne polyoxyéthylène ;
m représente 1 ;
m' représente 1 ou 2 ;
X' est choisi parmi les groupements octyl, décyl, dodécyl, $CF_3(CF_2)_rCH_2CH_2$-
$8 \geq r \geq 6$.

6. Procédé de préparation d'un composé répondant à la formule (I) selon l'une quelconque des revendications 1 à 5, ce procédé étant **caractérisé en ce que** l'on fait réagir un aldéhyde répondant à la formule (II) avec une hydroxylamine répondant à la formule (III) suivant le schéma 2 ci-dessous :

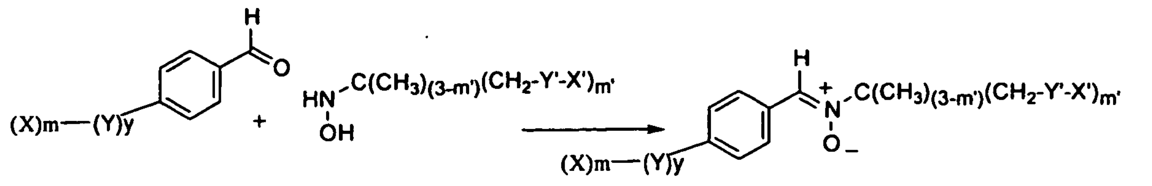

(II) (III) (I)

Schéma 2

7. Procédé selon la revendication 6, **caractérisé en ce que** le composé de formule (III) est préparé suivant un procédé décrit dans le schéma 3 :

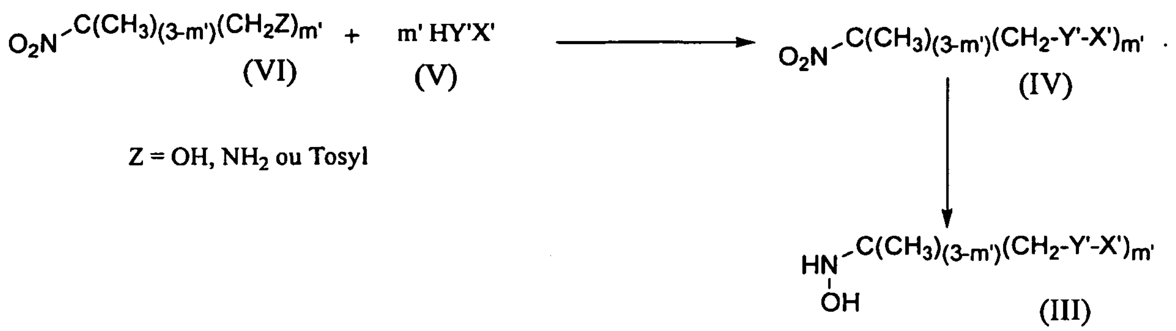


(VI) (V) (IV) (III)

Schéma 3

8. Composition pharmaceutique comprenant au moins un composé répondant à la formule (I) selon l'une quelconque des revendications 1 à 5 dans un support pharmaceutiquement acceptable.

9. Utilisation d'un composé répondant à la formule (I) selon l'une quelconque des revendications 1 à 5 pour la préparation d'un médicament destiné à prévenir et/ou traiter les effets des radicaux libres.

10. Utilisation d'un composé selon l'une quelconque des revendications 1 à 5 pour la préparation d'un médicament destiné à la prévention ou au traitement des pathologies liées au stress oxydatif et à la formation des espèces radicalaires oxygénées.

11. Utilisation selon la revendication 10, pour la prévention ou le traitement d'une pathologie choisie parmi les maladies immunitaires et inflammatoires, le syndrome d'ischémie-reperfusion, l'athérosclérose, la maladie d'Alzeimer, la maladie de Parkinson, les lésions dues aux radiations UV et ionisantes, la maladie de Huntington, les cancers, le vieillissement cellulaire.

12. Composition cosmétique, **caractérisée en ce qu'**elle comprend au moins un composé répondant à la formule (I) selon l'une quelconque des revendications 1 à 5 dans un support cosmétiquement acceptable.

13. Procédé de traitement cosmétique pour prévenir et/ou traiter les effets du vieillissement, **caractérisé en ce que**

l’on applique sur la peau ou sur les phanères une composition selon la revendication 12.

**14.** Utilisation d’un composé répondant à la formule (I) selon l’une quelconque des revendications 1 à 5, en synthèse organique comme capteur de radicaux libres dans des réactions radicalaires.

## Claims

**1.** Compound, **characterized in that** it has formula (I):

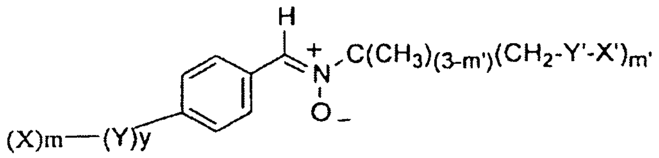

(I)

in which:

X is a hydrophilic group selected from mono- and polysaccharides as well as amino derivatives of mono- and polysaccharides, a polyethylene oxide chain and a peptide chain, or an ionic polar group selected from a quaternary ammonium group, an amine oxide and a carnitine group;
m is an integer equal to 1, 2 or 3;
Y is a spacer arm for linking the aromatic ring to the hydrophilic substituents X;
Y is selected from ester, amide, urea, urethane, ether, thioether and amine groups, and $C_1$-$C_6$ hydrocarbon chains optionally interrupted by one or more ester, amide, urea or urethane groups and by one or more ether, amine or thioether bridges;
y is an integer equal to 0 or 1;
Y’ is a group selected from an ester group, an amide group, a urea group, a urethane group, an ether bridge and a thioether bridge;
m’ is an integer selected from 1 and 2; and
X’ is a $C_4$-$C_{14}$ alkyl chain optionally substituted by one or more fluorine atoms.

**2.** Compound according to Claim 1, **characterized in that** X is a group selected from glucose, lactose, fructose, mannose, galactose, ribose, maltose, glucosamine, sucrose and lactobionamide.

**3.** Compound according to Claim 1, **characterized in that** X is a group selected from polyethylene oxide chains comprising from 30 to 100 ethylene oxide units, preferably from 50 to 60 units.

**4.** Compound according to Claim 1, **characterized in that** X is a group selected from

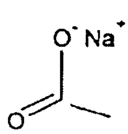 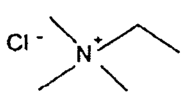 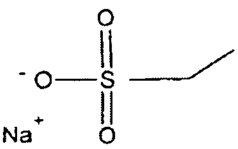

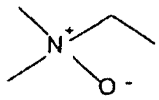

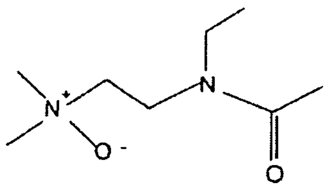

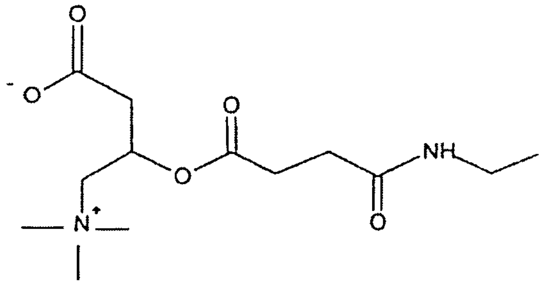

5. Compound according to Claim 1, **characterized in that** at least one of the following conditions is satisfied:

X is a group selected from lactobionamide, carnitine and a polyoxyethylene chain;
m is 1;
m' is 1 or 2; and
X' is selected from octyl, decyl and dodecyl groups and $CF_3(CF_2)_rCH_2CH_2$-, where $8 \geq r \geq 6$.

6. Process for the preparation of a compound of formula (I) according to any one of Claims 1 to 5, **characterized in that** an aldehyde of formula (II) is reacted with a hydroxylamine of formula (III) according to Scheme 2 below:

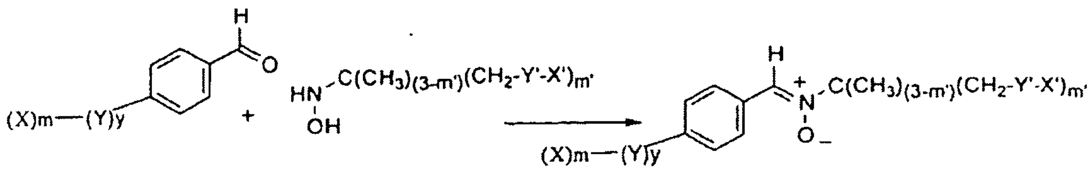

(II) (III) (I)

Scheme 2

7. Process according to Claim 6, **characterized in that** the compound of formula (III) is prepared by a process described in Scheme 3:

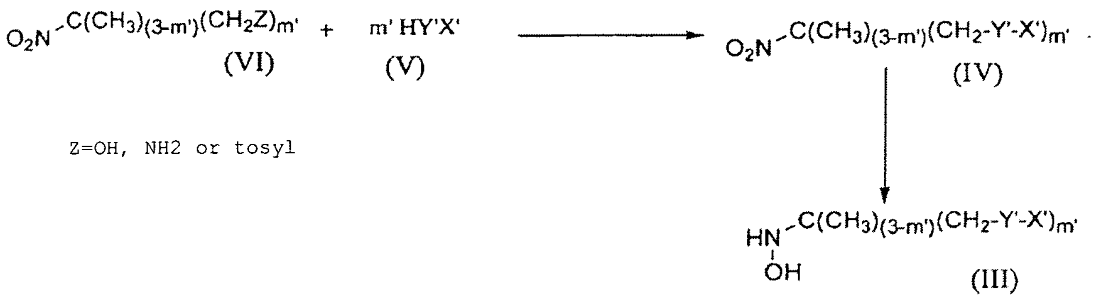


Scheme 3

8. Pharmaceutical composition comprising at least one compound of formula (I) according to any one of Claims 1 to 5 in a pharmaceutically acceptable excipient.

9. Use of a compound of formula (I) according to any one of Claims 1 to 5 for the preparation of a drug for preventing and/or treating the effects of free radicals.

10. Use of a compound according to any one of Claims 1 to 5 for the preparation of a drug for preventing or treating pathological conditions associated with oxidative stress and with the formation of oxygenated free radical species.

11. Use according to Claim 10 for preventing or treating a pathological condition selected from immune and inflammatory diseases, ischaemia-reperfusion syndrome, atherosclerosis, Alzheimer's disease, Parkinson's disease, lesions due to UV radiation and ionizing radiation, Huntington's disease, cancers and cellular ageing.

12. Cosmetic composition, **characterized in that** it comprises at least one compound of formula (I) according to any one of Claims 1 to 5 in a cosmetically acceptable excipient.

13. Method of cosmetic treatment for preventing and/or treating the effects of ageing, **characterized in that** a composition according to Claim 12 is applied to the skin or other superficial body growths.

14. Use of a compound of formula (I) according to any one of Claims 1 to 5 in organic synthesis as a free radical scavenger in free radical reactions.

**Patentansprüche**

1. Verbindung, **dadurch gekennzeichnet, dass** sie der Formel (I) entspricht:

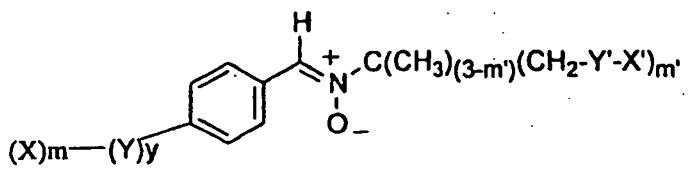

(I)

worin:

X eine hydrophile Gruppe bedeutet, die ausgewählt ist aus einem Mono- oder einem Polysaccharid sowie Aminoderivaten von Mono- und Polysacchariden, einer Polyethylenoxidkette, einer Peptidkette und einer polaren ionischen Gruppe, die ausgewählt ist aus quartärem Ammonium, einem Aminoxid und einer Carnitingruppe;
m eine ganze Zahl von 1, 2 oder 3 bedeutet;

Y einen Spacerarm zur Verknüpfung des aromatischen Kerns und der hydrophilen X-Substituenten bedeutet;
Y ausgewählt ist aus Ester-, Amid-, Harnstoff-, Urethan-, Ether-, Thioether- und Aminfunktionen, $C_1$-$C_6$-Kohlenwasserstoffketten, die gegebenenfalls durch ein oder mehrere Ester-, Amid-, Harnstoff- und Urethanfunktionen und ein oder mehrere Ether-, Amin- oder Thioetherbrücken unterbrochen sind;
y eine ganze Zahl von 0 oder 1 bedeutet;
Y' eine Gruppe bedeutet, die ausgewählt ist aus einer Esterfunktion, einer Amidfunktion, einer Harnstofffunktion, einer Urethanfunktion, einer Etherbrücke und einer Thioetherbrücke;
m' eine ganze Zahl ist, die ausgewählt ist aus 1 und 2;
X' eine gegebenenfalls mit ein oder mehr Fluoratomen substituierte $C_4$-$C_{14}$-Alkylkette bedeutet.

2. Verbindung nach Anspruch 1, **dadurch gekennzeichnet, dass** X eine Gruppe bedeutet, die ausgewählt ist aus: Glucose, Lactose, Fructose, Mannose, Galaktose, Ribose, Maltose, Glucosamin, Saccharose und Lactobionamid.

3. Verbindung nach Anspruch 1, **dadurch gekennzeichnet, dass** X eine Gruppe bedeutet, die ausgewählt ist aus Polyethylenoxidketten, die 30 bis 100, vorzugsweise 50 bis 60 Ethylenoxideinheiten umfassen.

4. Verbindung nach Anspruch 1, **dadurch gekennzeichnet, dass** X eine Gruppe bedeutet, die ausgewählt ist aus

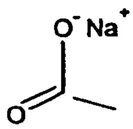
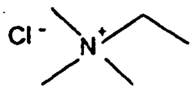
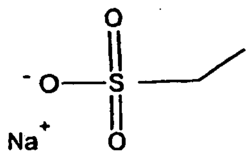

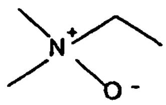
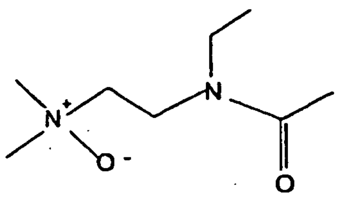

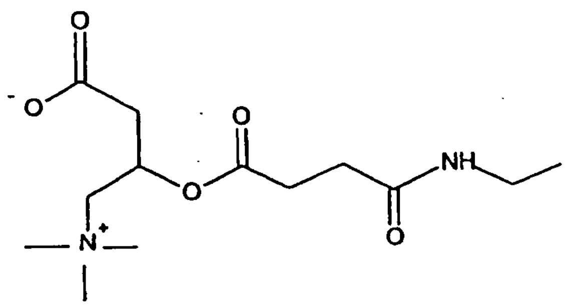

5. Verbindung nach Anspruch 1, **dadurch gekennzeichnet, dass** wenigstens eine der folgenden Bedingungen erfüllt ist:

X bedeutet eine Gruppe, die ausgewählt ist aus: Lactobionamid, Carnitin oder einer Polyoxyethylenkette;
m bedeutet 1;
m' bedeutet 1 oder 2;
X' ist ausgewählt aus den Gruppen Octyl, Decyl, Dodecyl und $CF_3(CF_2)_rCH_2CH_2$-, wobei $8 \geq r \geq 6$.

6. Verfahren zur Herstellung einer Verbindung der Formel (I) nach einem der Ansprüche 1 bis 5, wobei das Verfahren **dadurch gekennzeichnet ist, dass** ein Aldehyd der Formel (II) mit einem Hydroxylamin der Formel (III) entsprechend dem nachfolgenden Schema 2 umgesetzt wird:

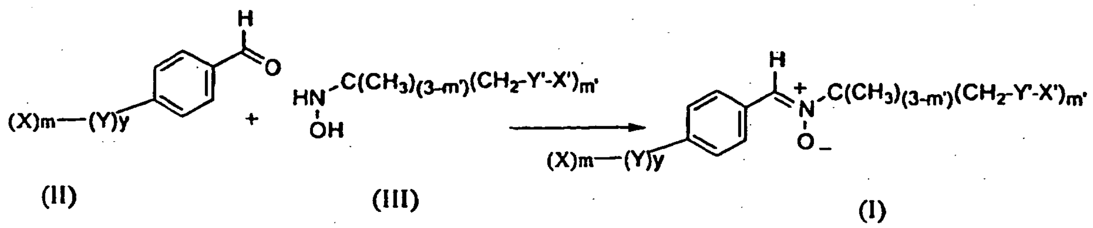

Schema 2

7. Verfahren nach Anspruch 6, **dadurch gekennzeichnet, dass** die Verbindung der Formel (III) nach einem in Schema 3 gezeigten Verfahren hergestellt wird:

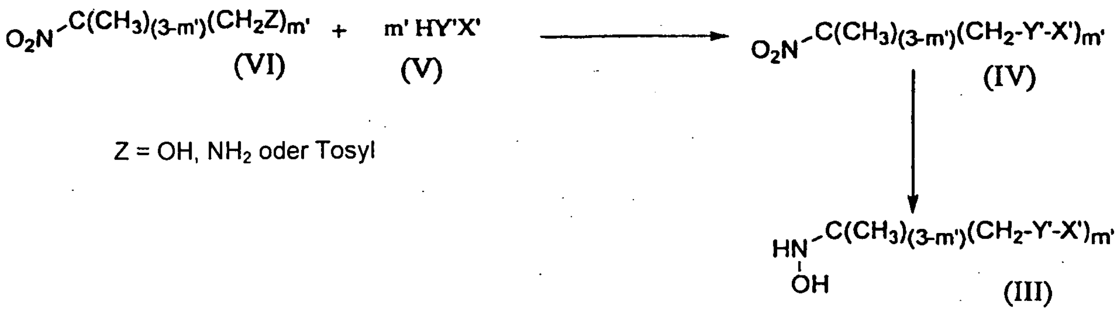

Schema 3

8. Pharmazeutische Zusammensetzung, umfassend wenigstens eine Verbindung der Formel (I) nach einem der Ansprüche 1 bis 5 in einem pharmazeutisch verträglichen Träger.

9. Verwendung einer Verbindung der Formel (I) nach einem der Ansprüche 1 bis 5 zur Herstellung eines Medikaments zur Vorbeugung und/oder Behandlung von Wirkungen freier Radikale.

10. Verwendung einer Verbindung nach einem der Ansprüche 1 bis 5 zur Herstellung eines Medikaments zur Vorbeugung oder Behandlung von pathologischen Zuständen in Verbindung mit oxidativem Stress und der Bildung sauerstoffhaltiger radikalischer Spezies.

11. Verwendung nach Anspruch 10 zur Vorbeugung oder Behandlung eines pathologischen Zustands, ausgewählt aus Immunkrankheiten und entzündlichen Krankheiten, Ischämie-Reperfusionssyndrom, Atherosklerose, Alzheimer-Krankheit, Parkinson-Krankheit, Läsionen als Folge von UV-Strahlung und ionisierender Strahlung, Huntington-Krankheit, Krebs und Zellalterung.

12. Kosmetische Zusammensetzung, **dadurch gekennzeichnet, dass** sie wenigstens eine Verbindung der Formel (I) nach einem der Ansprüche 1 bis 5 in einem kosmetisch verträglichen Träger umfasst.

13. Kosmetisches Behandlungsverfahren zur Vorbeugung und/oder Behandlung von Alterungseffekten, **dadurch gekennzeichnet, dass** man auf die Haut oder die Anhangsgebilde eine Zusammensetzung nach Anspruch 12 aufbringt.

14. Verwendung einer Verbindung der Formel (I) nach einem der Ansprüche 1 bis 5 bei der organischen Synthese als Fänger von freien Radikalen bei Radikalreaktionen.

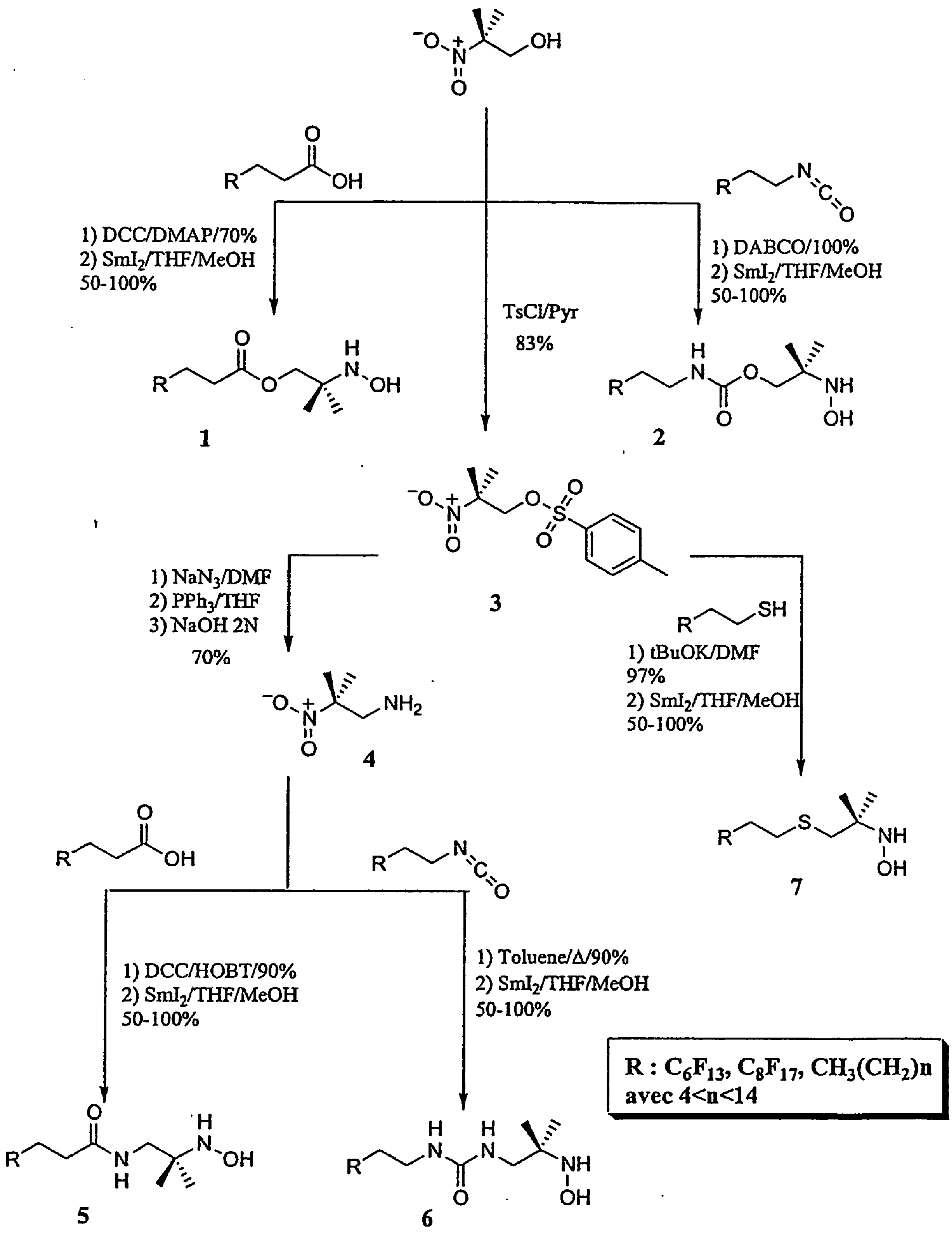


**Figure 1** : synthèse des parties hydrophobes monocaténaires hydro et perfluorocarbonées

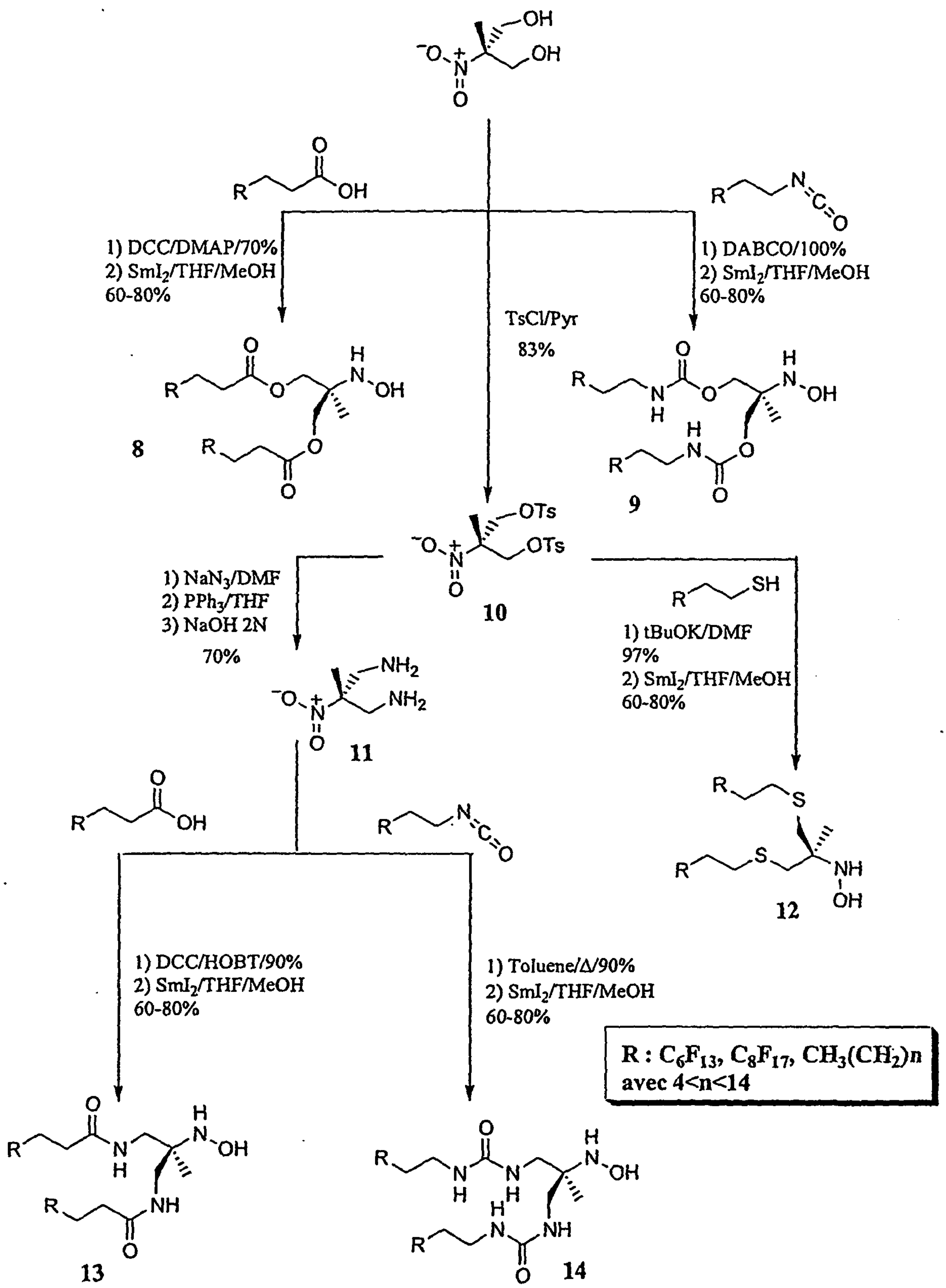


**Figure 2** : synthèse des parties hydrophobes bicaténaires hydro et perfluorocarbonées

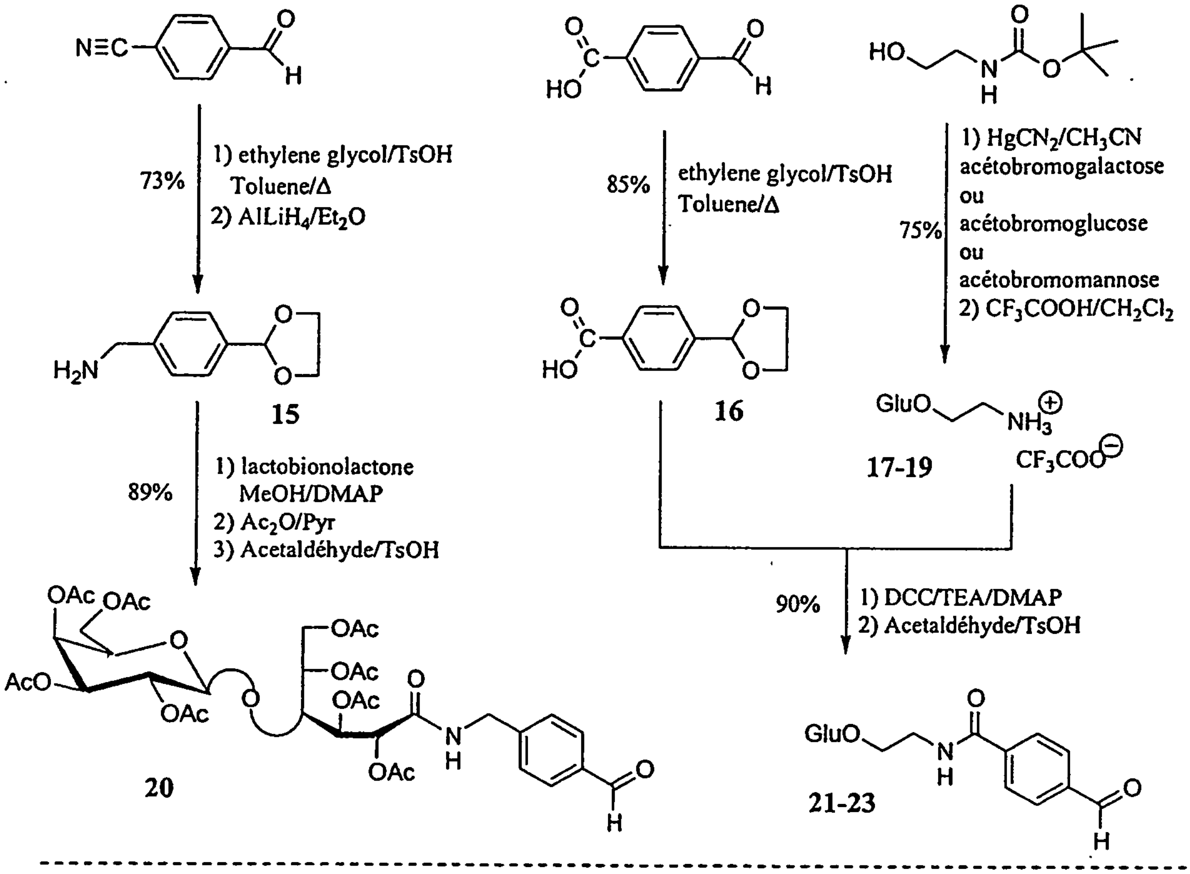


15

16

17-19

20

21-23

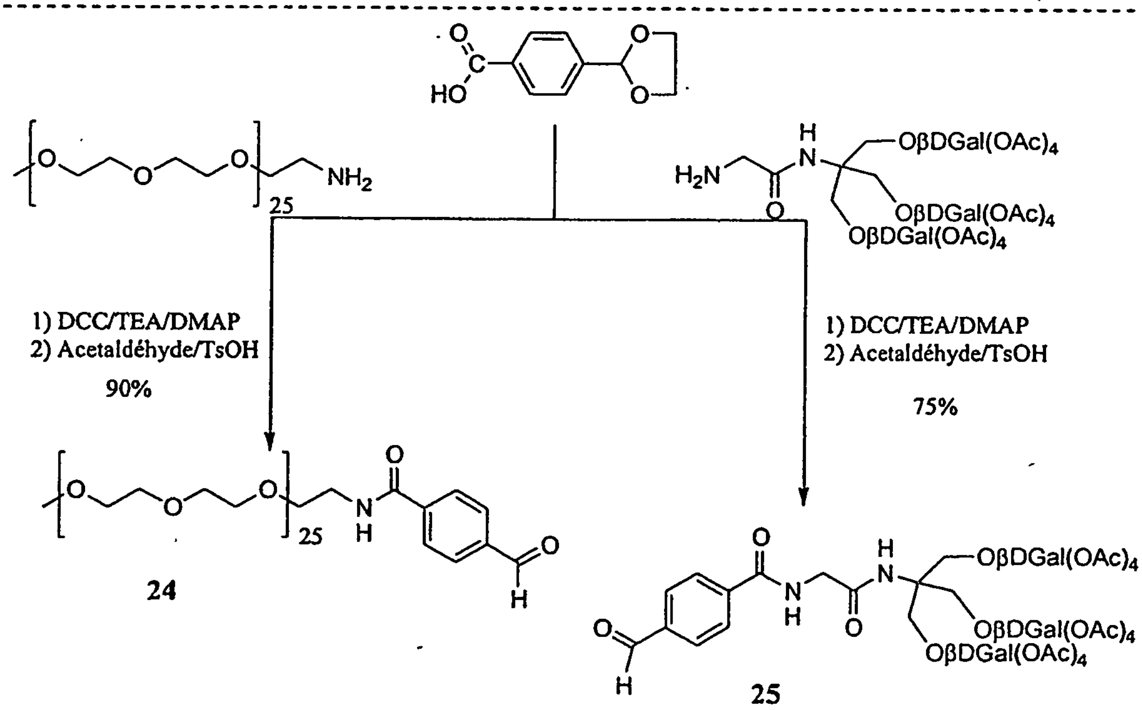


24

25

**Figure 3** : synthèse des têtes polaires non ioniques

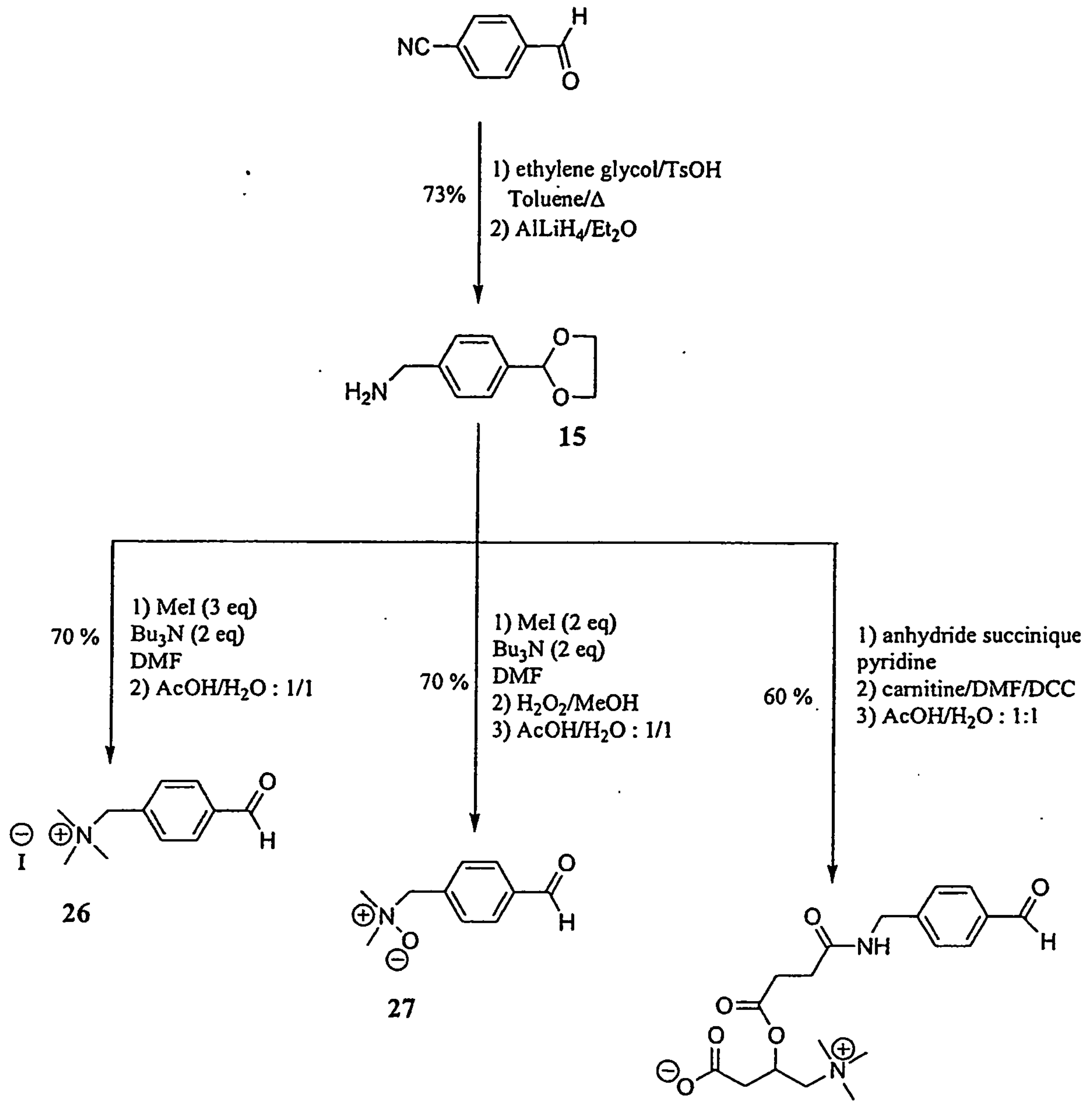


15

26

27

28

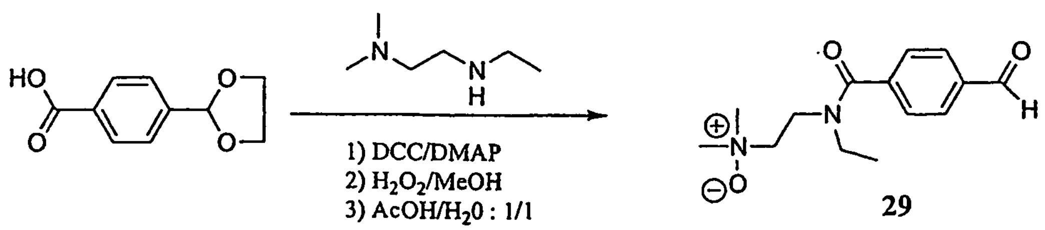


29

**Figure 4 :** synthèse des têtes polaires ioniques

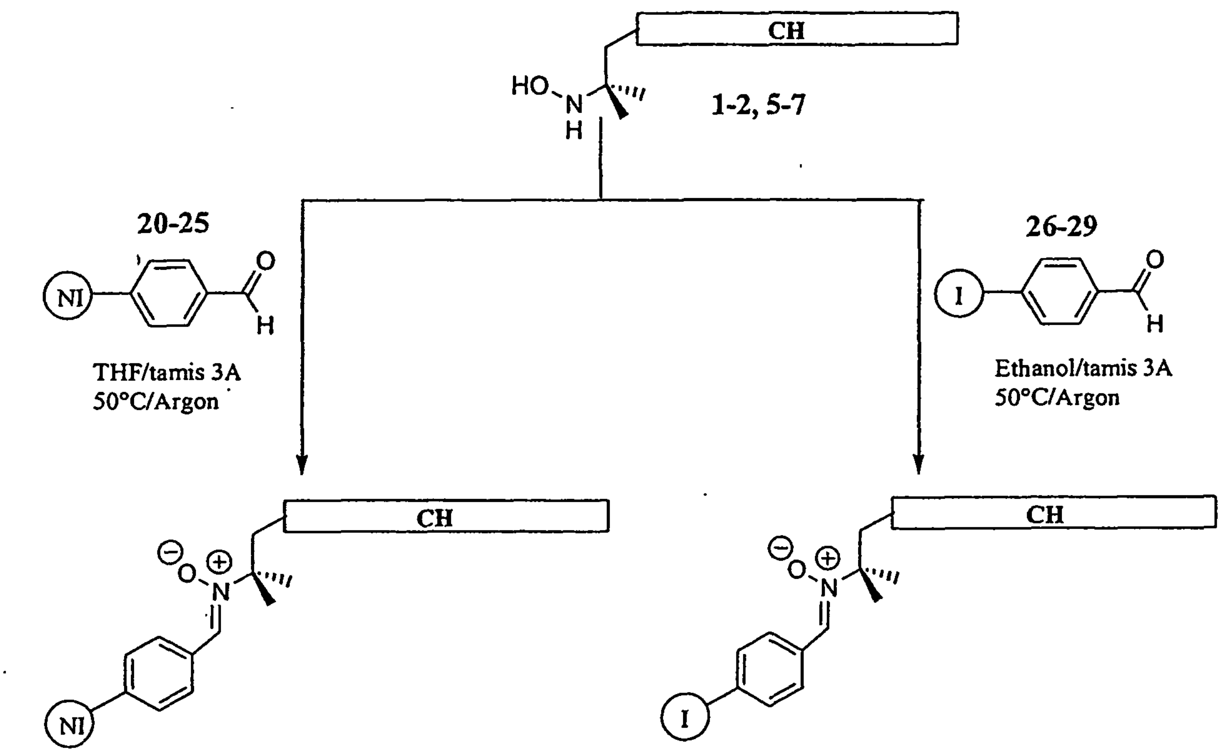


**A : dérivés amphiphiles monocaténaires non ioniques**

**B : dérivés amphiphiles monocaténaires ioniques**

**Figure 5 :** synthèse des nitrones amphiphiles monocaténaires **A-B**

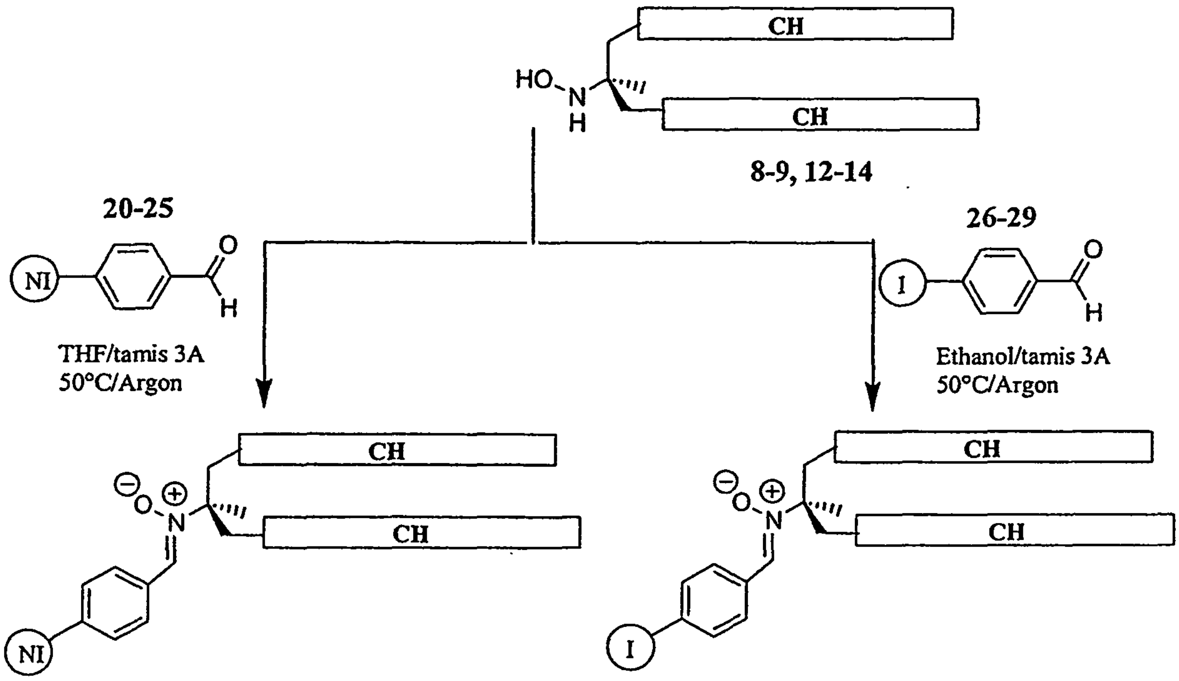


**C : dérivés amphiphiles bicaténaires non ioniques**

**D : dérivés amphiphiles bicaténaires ioniques**

**Figure 6 :** synthèse des nitrones amphiphiles bicaténaires **C-D**

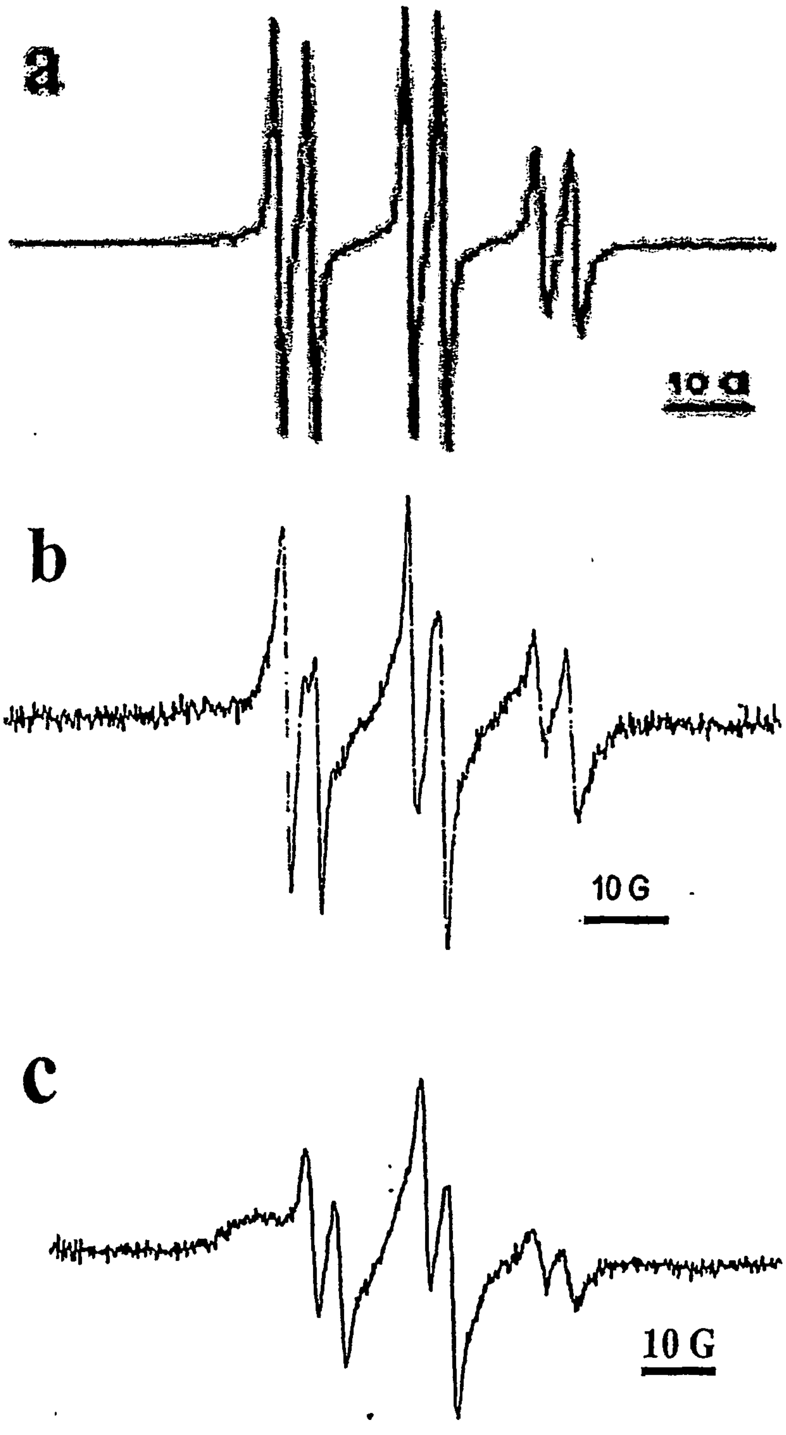


**Figure 7 :** Spectres RPE des adduits carboxylate (a), hydroxyle (b) et méthyle (c) générés respectivement par la réaction de Fenton (b) en présence de formiate (a) et de DMSO (c) et du composé $A_1$

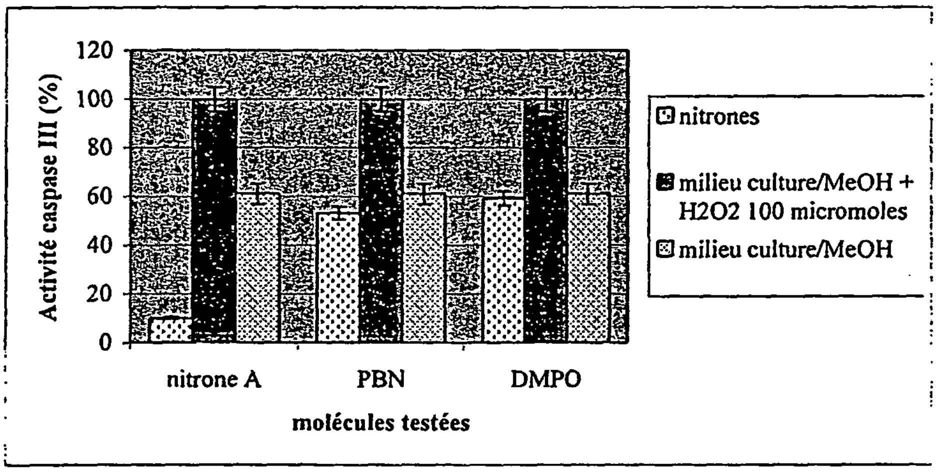


**Figure 8** : activité caspase III de cellules neuronales intoxiquées par $H_2O_2$ et traitées par des nitrones commerciales et la nitrone de type **A2**

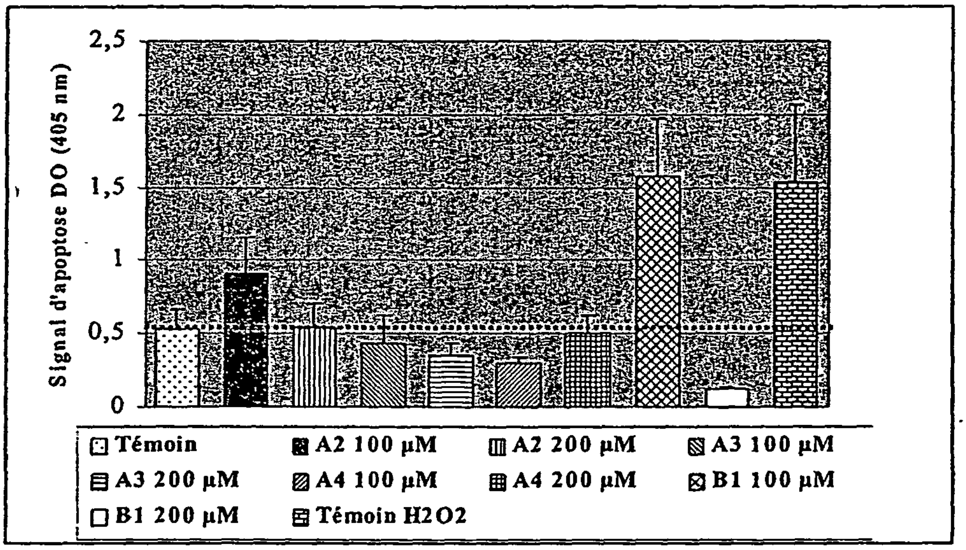


**Figure 9 :** Mesure de l'état d'apoptose par dosage ELISA de la fragmentation de l'ADN après lyse des cellules

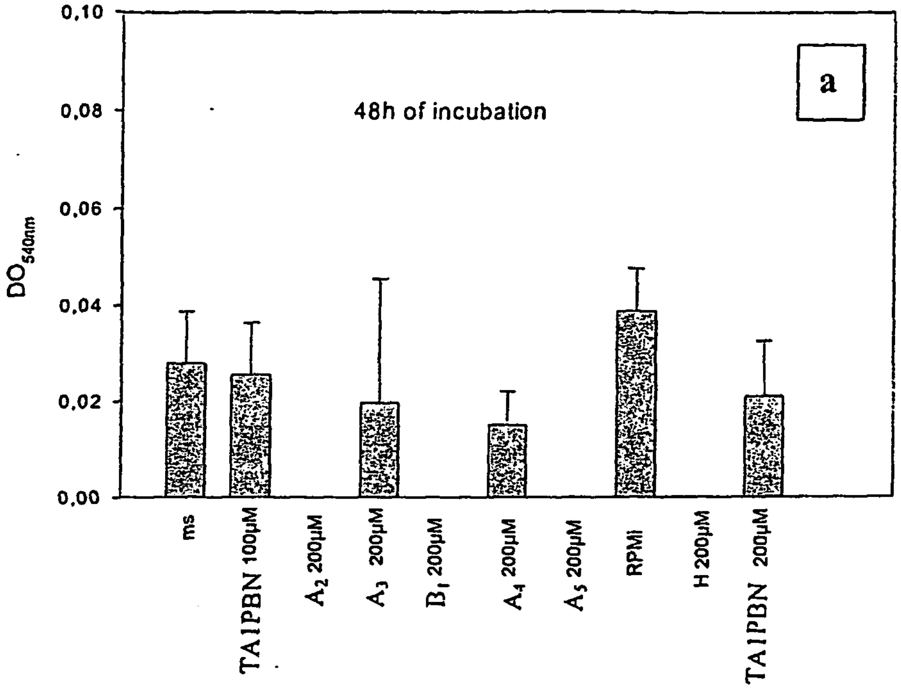


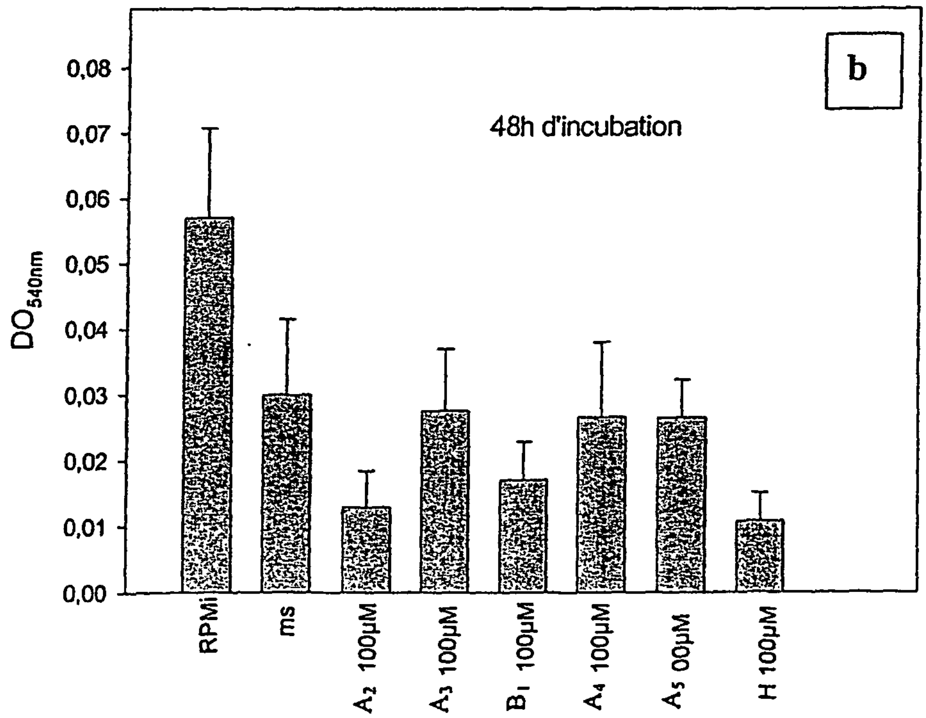


**Figures 10a et 10b** : Culture cellulaire NARP en présence de nitrones amphiphiles à 100 (a) et 200 µM (b) après 48h00 d'incubation

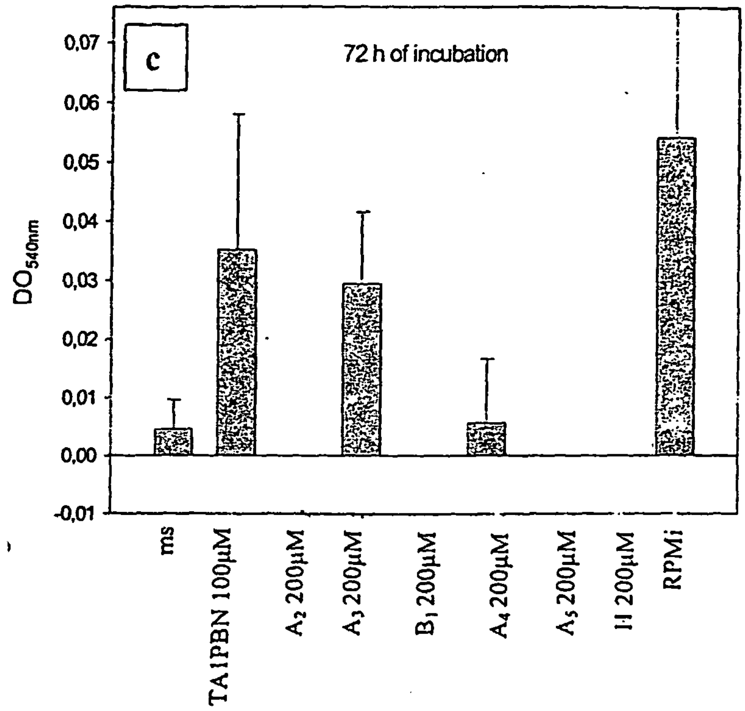


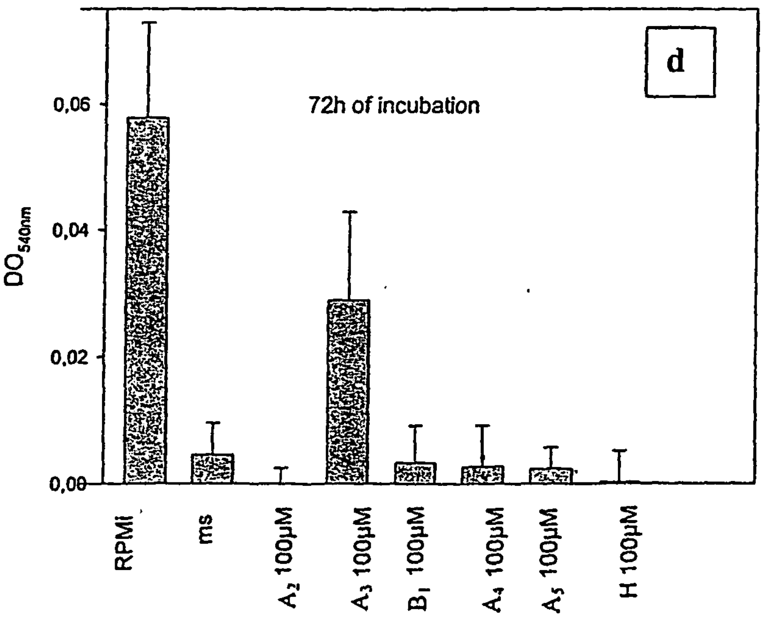


**Figures 10c et 10d :** Culture cellulaire NARP en présence de nitrones amphiphiles à 100 (d) et 200 µM (c) après 72h00 d'incubation

e

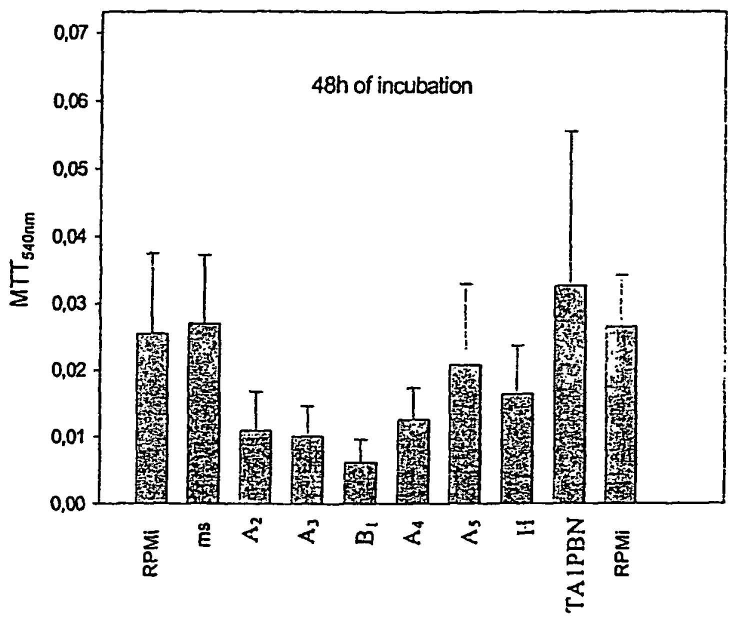


f

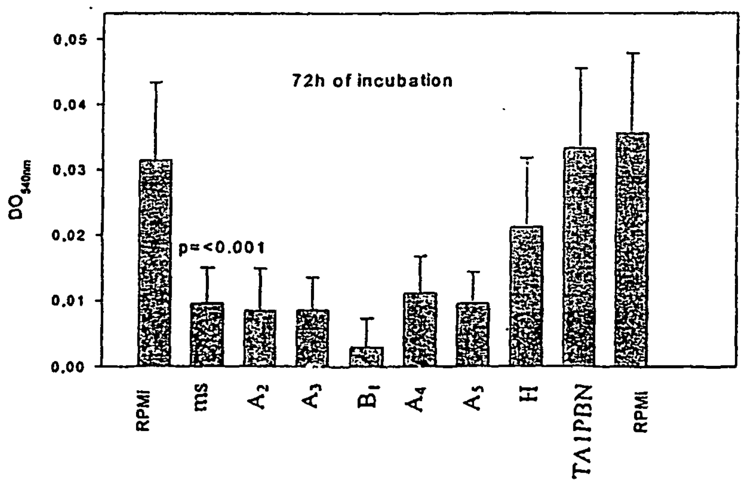


**Figures 10e et 10f** : Culture cellulaire NARP en présence de nitrones amphiphiles à 50 µM après 48h00 (e) et 72h00 (f) d'incubation

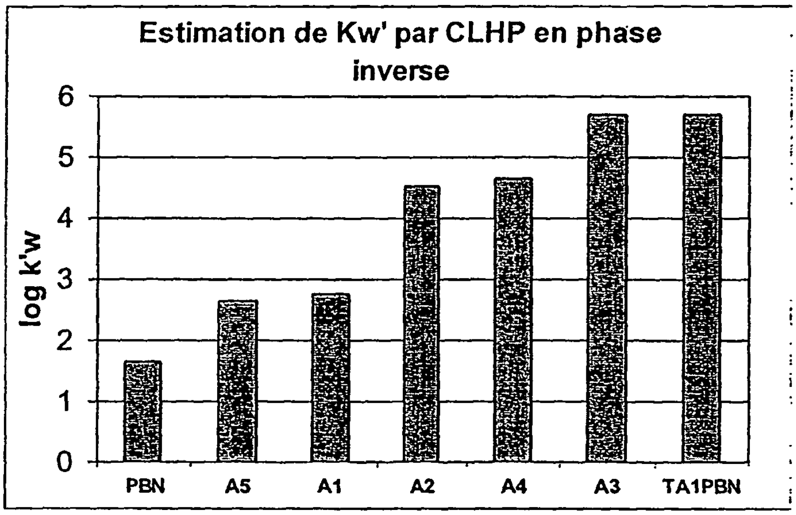


**Figure 11**: Variation de l'hydrophobie des nitrones dérivés de l'acide lactobionique **A1-A5**.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **Croos C.E.** *Arch. Intern. Med.,* 1987, vol. 107, 526-545
- **Anderson K.M. ; Ells G. ; Bonomi P. ; Harris J.E.** *Medical Hypotheses,* 1999, vol. 52, 53-57
- **Stadtman H.R. ; Berlett B.S.** *J. Biol. Chem.,* 1991, vol. 266, 17201-17211
- **Floyd R.A.** *Carcinogenesis,* 1990, vol. 11, 1447-1450
- **Gille J.J. ; Van Berkel C.G. ; Joenge H.** *Carcinogenesis,* 1994, vol. 15, 2695-2699
- **Halliwell B.** *Mutat. Res.,* 1999, vol. 443, 37-52
- **Siebenlist U. ; Franzoso G. ; Brown K.** *Annu. Rev. Cell. Biol.,* 1994, vol. 10, 405-455
- **Oliver C. ; Starke-Read P. ; Stadman E. ; Liu G. ; Carney J. ; Floyd R.** *Proc. Natl. Acad. Sci. USA,* 1990, vol. 87, 5144-5147
- **Hensley K. ; Carney J.M. ; Stewart C.A. ; Tabatabaie T. ; Pye Q.N. ; Floyd R.A.** *Int. Rev. Neurobiol.,* 1997, vol. 40, 229-317
- **Cheng H.Y. ; Liu T. ; euerstein G. ; Barone F.C.** *Free Radic. Biol. Med,* 1993, vol. 14, 243-250
- **Kuroda S. ; Tsuchidate R. ; Smith M.L. ; Maples K.R. ; Siesjo B.K.** *J. Cereb. Blood Flow Metab.,* 1999, vol. 19, 778-787
- **Lees K.R. ; Sharma A.K. ; Barer D. ; Ford G.A. ; Kostulas V. ; Cheng Y.F. ; Odergren T.** *Stroke,* 2001, vol. 32, 675-680
- **Almli L.M. ; Hamrick S.E.G. ; Koshy A.A. ; Täuber M.G. ; Ferriero D.M.** *Dev. Brain Res.,* 2001, vol. 132, 121-129
- **Nakao N ; Grasbon-Frodl E.M. ; Widner H. ; Brundin P.** *Neuroscience,* 1996, vol. 73, 185-200
- **Ouari O. ; Polidori A. ; Pucci B. ; Tordo P. ; Chalier F.** *J. Org. Chem.,* 1999, vol. 64, 3554-3556
- **Geromel V. ; Kadhom N. ; Cebalos-Picot I. ; Ouari O. ; Polidori A. ; Munnich A. ; Rötig A. ; Rustin P.** *Hum. Mol. Genet.,* 2001, vol. 10, 1221-1228
- **Girard P. ; Namy J.L. ; Kagan H.B.** *J. Am. Chem. Soc.,* 1980, vol. 102, 2693-2698
- **Namy J.L. ; Girard P ; Kagan H.B.** *Nouv. J. Chem.,* 1977, vol. 1, 5
- **Polidori A. ; Pucci B. ; Zarif L. ; Lacombe J-M. ; Riess J-G. ; Pavia A.A.** *Chem. Phys. Lipids,* 1995, vol. 77, 225-251
- **Sommer H.Z. ; Lipp H.I ; Jackson L.L.** *J. Org. Chem.,* 1971, vol. 36, 824-828
- **McQuade D.T. ; Quinn M.A. ; YU S.M. ; Polans A.S. ; Krebs M.P. ; Gellman S.H.** *Angew. Chem. Int. Ed.,* 2000, vol. 39, 758-761
- **Whittemore E.R. ; Loo D.T. ; Cotman C.W.** *Neuroreport,* 1994, vol. 5, 1485-1488
- **Nicholson D.W. ; Ali A. ; Thombury N.A. ; Vaillancourt J.P. ; Ding C.H. ; Gallant M. ; Griffm P.R. ; Labelle M. ; Lazebnik Y.A. ; Munday N.A.** *Nature,* 1995, vol. 376, 37-43
- **Ouari O. ; Chalier F. ; Pucci B. ; Tordo P.** *J. Chem. Soc., Perkin Trans,* 1998, vol. 2, 2299
- **Lambert, W. J.** *J. Chromtogr.,* 1993, vol. 656, 469
- **Dorsey, J. G. ; Kahaledi, M. G.** *J. Chromtogr.,* 1993, vol. 656, 485
- **Fevig, T. L. ; Bowen, S. M. ; Janowick, D. A. ; Jones, B. K. ; Munson, H. R. ; Ohlweiler, D. F. ; Thomas, C. E.** *J. Med Chem.,* 1996, vol. 39, 4988